# EUROPEAN PATENT SPECIFICATION

(11) **EP 3 313 988 B1**
(45) Date of publication and mention of the grant of the patent: **06.03.2024**
(21) Application number: 16738208.4
(22) Date of filing: 23.06.2016
(51) Int. Cl.: C12N 9/02, C12N 15/82

(54) **PRODUCTION OF NOSCAPINE**
HERSTELLUNG VON NOSCAPIN
PRODUCTION DE NOSCAPINE

(30) Priority: 24.06.2015 GB 201511156
(43) Date of publication of application: 02.05.2018
(73) Proprietor: Sun Pharmaceutical Industries Australia Limited, Notting Hill, Victoria 3168 (AU); The University Of York, York, Yorkshire YO10 5DD (GB)
(72) Inventor: WINZER, Thilo, York Yorkshire YO10 5DD (GB); GRAHAM, Ian, York Yorkshire YO10 5DD (GB); WALKER, Tracy, Wilsonton Queensland 4350 (AU)
(74) Representative: Symbiosis IP Limited
(86) International application number: PCT/GB2016/051887
(87) International publication number: WO 2016/207643

(56) References cited:
- WO-A1-2015/021561
- WO-A1-2015/081437
- WO-A1-2015/173590
- T. WINZER ET AL: "Morphinan biosynthesis in opium poppy requires a P450-oxidoreductase fusion protein", SCIENCE, vol. 349, no. 6245, 17 July 2015 (2015-07-17), pages 309-312, XP055298478, US ISSN: 0036-8075, DOI: 10.1126/science.aab1852
- FUMIHIKO SATO: "Improved Production of Plant Isoquinoline Alkaloids by Metabolic Engineering", ADVANCES IN BOTANICAL RESEARCH, LONDON, GB, vol. 68, 1 January 2013 (2013-01-01), pages 163-181, XP009191456, ISSN: 0065-2296

## Description

### Field of the Invention

This disclosure relates to high noscapine producing plants comprisingfunctional, or a non-functional or partially functional copy of a cytochrome P450-oxidoreductase catalysing the conversion of (S)-reticuline to (R)-reticuline in plants of the genus *Papaver.*

### Background to the Disclosure

*Papaver somniferum,* the opium poppy, is a source of clinically useful opiate alkaloids such as morphine, codeine, thebaine, noscapine and papaverine. Opiate alkaloids have broad clinical application ranging from analgesics to cough suppressants and anti-spasmodics and are known for inducing physical dependencies.

Opiate alkaloids are structurally diverse with benzylisoquinoline providing the structural backbone and include compounds such as papaverine, noscapine, codeine, apomorphine, bernerine and sanguinarine. Benzylisoquinoline alkaloids can be further grouped based on their chemical structure into morphinans and phthalideisoquinoline alkaloids. Morphine, codeine, and thebaine are examples of morphinans, whereas papaverine and noscapine belong to the class of phthalideisoquinolines. Unlike morphinans noscapine has no analgesic properties but found its application as cough suppressant and acts as a potent antitumor agent binding to tubulin, inhibiting its polymerisation, arresting cell division and inducing apoptosis. The anti-tumor activity of noscapine and its derivatives was shown in various forms of cancer. Interestingly, contrary to most other opiate alkaloids, noscapine does not induce physical dependencies.

Benzylisoquinoline alkaloids are found in a number of species in the Papaveraceae family, but morphinan production has only been reported in opium poppy and the closely related *P. setigerum.* Total chemical synthesis, although possible, is not an economically viable means of production. Consequently, morphinan alkaloids are still exclusively sourced from the opium poppy plant. Whilst the biosynthesis of morphinans has been extensively studied over the last 25 years and the majority of genes in the pathway have been identified, the genes involved in the first step of morphinan synthesis, the epimerization of (S)-to (R)-reticuline, required for the development of efficient and economic viable morphinan production in microbial based systems have just recently been discovered in *P. somniferum* and are disclosed in patent application PCT/GB2015/051446 (WO2015173590). (S)-reticuline is transformed to (R)-reticuline via 1,2-Dehydroreticuline by a fusion protein encoding both a cytochrome P450 and oxidoreductase module, the latter belonging to the aldo-keto reductase family. Metabolite analysis of mutant alleles and heterologous expression demonstrate that the P450 module is responsible for the conversion of (S)-reticuline to 1,2-dehydroreticuline while the oxidoreductase module converts 1,2-dehydroreticuline to (R)-reticuline rather than functioning as a P450 redox partner. A similar fusion protein catalyzing the conversion from (S)- to (R)-reticuline has also been described in WO 2015/081437.

Likewise, noscapine synthesis has only been reported in a number of Papaver species. The level of noscapine in high noscapine varieties is typically less than 3 % of the capsule dry weight (which corresponds to a noscapine concentration of less than 60% of the total benzylisoquinoline alkaloid content) and relatively low and when compared to the morphinan levels which constitute over 41 % of the capsule dry weight. WO2009/143574 and WO2009/109012 discloses *P*. *somniferum* with noscapine concentrations based on the alkaloid concentration in poppy straw of between 0.1 to a maximum of 3.1 %. The production of noscapine was found to require the presence of a complex cluster of co-expressed genes comprising mostly cytochrome P450 and methyltransferases converting scoulerine via several steps to noscapine and is disclosed in US2015/0004659.

This disclosure relates to the identification and characterization of high noscapine opium poppy plants comprising a non-functional version of the P450-oxidoreductase fusion protein involved in the conversion of (S) to (R)-reticuline. Plants having the mutated gene have enhanced noscapine concentrations of over 3 % noscapine of the capsule dry weight (over at least 50% noscapine of the total benzylisoquinoline alkaloid content). Poppy plants comprising the modified fusion gene or engineered plants that are either null or have reduced expression of the wild-type fusion gene provide an alternative, more efficient source of noscapine.

### Statements of Invention

According to an aspect of the invention there is provided a plant of the genus *Papaver* wherein said plant is modified in a gene comprising a modified nucleotide sequence as set forth in SEQ ID NO: 6, 7 or 8, or a nucleic acid molecule which is at least 90% identical to the sequence set forth in SEQ ID NO 6, 7 or 8 over the full nucleotide sequence and encodes a modified cytochrome P450-oxidoreductase catalysing the conversion of (*S*)-reticuline to (*R*)-reticuline wherein said modification is homozygous, stably inherited and reduces or abrogates the expression or activity of said cytochrome P450 domain and/or oxidoreductase domain of said modified polypeptide wherein noscapine comprises at least 50% of the benzylisoquinoline alkaloid content of said modified plant.

In a preferred embodiment of the invention said modified nucleic acid molecule is:
i) at least 91%, 92%, 93%, 94%, 95%, 96%, 97%, 98% or 99% identical to the nucleotide sequence set forth in SEQ ID NO: 6, 7 or 8 over the full nucleotide sequence; or
ii) is at least 99% identical to the nucleotide sequence set forth in SEQ ID NO 6, 7 or 8, over the full nucleotide sequence and wherein said modification is an addition, substitution or deletion of at least one nucleotide base between nucleotides 1280-1300 set forth in SEQ ID NO 6, 7 or 8.

In a preferred embodiment of the invention said modification is a substitution of at least one nucleotide base between nucleotides 1280-1300 set forth in SEQ ID NO 6, 7 or 8 or wherein said modification is a substitution of at least one nucleotide base between nucleotides 1285-1295 set forth in SEQ ID NO 6, 7 or 8 or wherein said modification is a C to T substitution of the nucleotide base at nucleotide 1291 of SEQ ID NO 6, 7 or 8.

In a preferred embodiment of the invention said modified polypeptide has reduced or abrogated activity and is selected from the group consisting of:
i) a polypeptide comprising a modified amino acid sequence of the amino acid sequence set forth in SEQ ID NO: 9, 10 or 11;
ii) a polypeptide comprising a modified amino acid sequence that is at least 95% identical to the full length amino acid sequence as set forth in SEQ ID NO: 9, 10 or 11, wherein said sequence is modified by addition, deletion or substitution of one or more amino acid sequences and wherein said polypeptide has reduced or abrogated enzyme activity in the conversion of (*S*)-reticuline to 1,2-dehydroreticuline and/or to (*R*)-reticuline when compared to the unmodified polypeptide comprising the amino acid sequence set forth in SEQ ID NO: 9, 10 or 11.

In a preferred embodiment of the invention said modified polypeptide has reduced or abrogated activity and is selected from the group consisting of:
i) a modified polypeptide comprising a modified amino acid sequence of the amino acid sequence set forth in SEQ ID NO: 15;
ii) a modified polypeptide comprising a modified amino acid sequence of the amino acid sequence set forth in SEQ ID NO: 16;
iii) a modified polypeptide comprising a modified amino acid sequence that is at least 95% identical to the full length amino acid sequence as set forth in SEQ ID NO: 15 or 16, wherein said sequence is modified by addition, deletion or substitution of one or more amino acid sequences and wherein said polypeptide has reduced or abrogated enzyme activity in the conversion of 1,2-dehydroreticuline to (*R*)-reticuline when compared to the unmodified polypeptide comprising the amino acid sequence set forth in SEQ ID NO: 15 or 16.

In a preferred embodiment of the inventio n said modified polypeptide is selected from the group:
i) at least an addition, deletion or substitution of an amino acid residue in the region of amino acids 386-486 as set forth in SEQ ID NO: 9, 10 or 11;
ii) at least an addition, deletion or substitution of an amino acid residue in the region of amino acids 426-436 as set forth in SEQ ID NO: 9, 10 or 11 and wherein said modified polypeptide is at least 90% identical to the amino acid sequence set forth in SEQ ID NO: 9, 10 or 11; or
iii) said modified polypeptide comprises a deletion or substitution of amino acid residue 431 of SEQ ID NO: 9 wherein said modified polypeptide is at least 90% identical to the amino acid sequence set forth in SEQ ID NO: 9.
iv) said modified polypeptide comprises a deletion or substitution of amino acid residue 432 of SEQ ID NO: 10 wherein said modified polypeptide is at least 90% identical to the amino acid sequence set forth in SEQ ID NO: 10;
v) said modified polypeptide comprises a deletion or substitution of amino acid residue 431 of SEQ ID NO: 11 wherein said modified polypeptide is at least 90% identical to the amino acid sequence set forth in SEQ ID NO: 11;
vi) said modified polypeptide comprises a substitution or deletion at amino acid arginine 431 of the amino acid sequence set forth in SEQ ID NO: 9;
vii) wherein said modified polypeptide comprises a substitution or deletion at amino acid arginine 432 of the amino acid sequence set forth in SEQ ID NO: 10;
viii) wherein said modified polypeptide comprises a substitution or deletion at amino acid arginine 431 of the amino acid sequence set forth in SEQ ID NO: 11;
ix) wherein said modification is the substitution of arginine for tryptophan in at position 431 of the amino acid sequences set forth in SEQ ID NO: 9;
x) wherein said modification is the substitution of arginine for tryptophan in at position 432 of the amino acid sequences set forth in SEQ ID NO: 10;
xi) wherein said modification is the substitution of arginine for tryptophan in at position 431 of the amino acid sequences set forth in SEQ ID NO: 11.

In a preferred embodiment of the invention said modified polypeptide comprises the amino acid sequence set forth in SEQ ID NO: 27, 28 or 29.

In a preferred embodiment of the invention said plant comprises one or more genes comprising a nucleotide sequence selected from the group consisting of:
i) a nucleotide sequence as represented by the sequence in SEQ ID NO: 17,18, 19, 20, 21, 22, 23, 24, 25 or 26;
ii) a nucleotide sequence wherein said sequence is degenerate as a result of the genetic code to the nucleotide sequence defined in (i);
iii) a nucleic acid molecule the complementary strand of which hybridizes under stringent hybridization conditions to the sequence in SEQ ID NO: 17,18, 19, 20, 21, 22, 23, 24, 25 or 26 wherein said nucleic acid molecule encodes polypeptides involved in the biosynthesis of *Papaver* noscapine or intermediates in the biosynthesis of noscapine.

In a preferred embodiment of the invention said plant is null for said cytochrome P450-oxidoreductase.

According to a further aspect of the invention there is provided a Papaver capsule, straw or seed obtained from the modified plant according to the invention.

According to a further aspect of the invention there is provided the use of a gene encoding a cytochrome P450-oxidoreductase catalysing the conversion of (*S*)-reticuline to (*R*)-reticuline in the identification of *Papaver* cultivars that have enhanced levels of noscapine or intermediates in noscapine synthesis according to the invention wherein said gene comprises a nucleotide sequence selected from the group consisting of:
i) a nucleic acid molecule comprising a nucleic acid sequence as set forth in SEQ ID NO: 6, 7 or 8; or
ii) a nucleic acid molecule the complementary strand of which hybridizes under stringent hybridization conditions to the nucleotide sequence set forth in SEQ ID NO: 6, 7 or, 8 and which encodes a polypeptide that has reduced or abrogated expression or activity of a cytochrome P450-oxidoreductase catalysing the conversion of (*S*)-reticuline to (*R*)-reticuline compared to a wild-type *Papaver* plant comprising a gene encoding a functional cytochrome P450-oxidoreductase.

According to an aspect of the invention there is provided a method to identify a *Papaver* plant comprising a modified cytochrome P450-oxidoreductase gene comprising the steps:
i) obtaining a sample of plant material from a plant to be tested;
ii) extracting genomic nucleic acid from said plant material; and
iii) analysing the extracted genomic nucleic acid encoding a variant nucleotide sequence of the nucleotide sequences set forth in SEQ ID NO: 6, 7 or 8; and optionally,
iv) comparing the sequence to a nucleotide sequence comprising SEQ ID NO: 6, 7 or 8; and optionally
v) preparing a reaction mixture comprising:
   a) genomic DNA to be analysed,
   b) a first allele specific forward oligonucleotide primer comprising a nucleotide sequence complementary to said cytochrome P450-oxidoreductase gene nucleotide sequence and a nucleotide sequence complementary to a nucleotide sequence of a first fluorescent reporter cassette,
   c) a second allele specific forward oligonucleotide primer comprising a nucleotide sequence complementary to said cytochrome P450-oxidoreductase gene an a nucleotide sequence complementary to a nucleotide sequence of a second fluorescent reporter cassette wherein the nucleotide at the 3'-end of said second oligonucleotide primer sequence is complimentary to a polymorphic nucleotide sequence variant of said cytochrome P450-oxidoreductase gene, and wherein the nucleotide sequence of said first and second oligonucleotide primers are complementary to the same upstream nucleotide sequence of said cytochrome P450-oxidoreductase gene;
   d) a reverse oligonucleotide primer comprising a nucleotide sequence complementary to a nucleotide sequence downstream of said cytochrome P450-oxidoreductase gene nucleotide sequence; and
   e) two reporter cassettes comprising double stranded nucleic acid comprising:
      a first double stranded nucleic acid wherein one strand comprises a fluorescent label and is complementary to said first allele specific primer and a second strand comprising a quencher label that quenches said fluorescent label; and
      a second double stranded nucleic acid wherein one strand comprises a fluorescent label different from the fluorescent label of i) and is complementary to said second allele specific primer and a second strand comprising a quencher label that quenches said second fluorescent label;
vi) heating said reaction mixture to denature genomic nucleic acid and said double stranded reporters and providing conditions to anneal primers to their respective complementary nucleotide sequence, primer extending said annealed oligonucleotide primers and polymerase chain amplification of said mixture; and
vii) detecting fluorescence emission.

Competitive allele specific PCR (KASP) allows the identification of single-nucleotide polymorphism in a DNA sequence. The method is based on primer extension of two allele-specific (forward) primers and amplification of the resulting product using a common reverse primer. The forward primers are designed specifically to bind and extend the primer to either the SN P or wild type allele which allows subsequent binding of the common reverse primer for amplification of the product. In a second step an oligonucleotide cassette complimentary to a specific 5' sequence of the first allele-specific forward primer and a second oligonucleotide cassette complimentary to a specific 5' sequence of the second allele-specific forward primer both comprising a different fluorescent label is added to the reaction allowing amplification of the SNP and wild type targets and product detection through fluorescent signalling. Alternatively, directly fluorescent labelled primers targeted to the common reverse primer can be used to amplify e.g. just the species with the SNP in a sample. The KASP technology is disclosed in US2016/0002712.

It will be apparent to the skilled artisan that techniques that enable the analysis of mutations in gene sequences and/or expression are available and include, but are not limited to genomic DNA sequencing, PCR based analysis of DNA via single polynucleotide polynucleotide polymorphisms (SNP analysis), single stranded conformational polymorphism (SSCP), denaturing gradient gel electrophoresis (DGGE), quantitative PCR and multiplex PCR to detect expression of multiple genes.

According to a further aspect of the invention there is provided a method for the extraction of noscapine from a modified plant or capsule or straw according to the invention comprising the steps:
i) harvesting a modified plant, capsule or straw according to the invention and forming a reaction mixture of particulate plant material;
ii) solvent extracting said reaction mixture to provide an alkaloid enriched fraction; and
concentrating said enriched alkaloid fraction to provide an enriched noscapine fraction.

Hybridization of a nucleic acid molecule occurs when two complementary nucleic acid molecules undergo an amount of hydrogen bonding to each other. Isolated nucleic acid molecules as referred herein include genomic DNA, cDNA molecules and RNA molecules. The stringency of hybridization can vary according to the environmental conditions surrounding the nucleic acids, the nature of the hybridization method, and the composition and length of the nucleic acid molecules used. Calculations regarding hybridization conditions required for attaining particular degrees of stringency are discussed in Sambrook et al., Molecular Cloning: A Laboratory Manual (Cold Spring Harbor Laboratory Press, Cold Spring Harbor, NY, 2001); and Tijssen, Laboratory Techniques in Biochemistry and Molecular Biology-Hybridization with Nucleic Acid Probes Part I, Chapter 2 (Elsevier, New York, 1993). The Tₘ is the temperature at which 50% of a given strand of a nucleic acid molecule is hybridized to its complementary strand. The following is an exemplary set of hybridization conditions and is not limiting:
Very High Stringency (allows sequences that share at least 90% identity to hybridize)

| | |
|---|---|
| Hybridization: | 5x SSC at 65°C for 16 hours |
| Wash twice: | 2x SSC at room temperature (RT) for 15 minutes each |
| Wash twice: | 0.5x SSC at 65°C for 20 minutes each |

High Stringency (allows sequences that share at least 80% identity to hybridize)

| | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |

Low Stringency (allows sequences that share at least 50% identity to hybridize)

| | |
|---|---|
| Hybridization: | 6x SSC at RT to 55°C for 16-20 hours |
| Wash at least twice: | 2x-3x SSC at RT to 55°C for 20-30 minutes each |

In a preferred embodiment of the invention there is provided a modified polypeptide encoded by nucleic acid molecule comprising a nucleotide sequence as herein disclosed.

In an embodiment of the invention said plant expresses a modified P450-oxidoreductase polypeptide wherein said modified polypeptide has reduced or abrogated activity in the conversion of (*S*)-reticuline to (*R*)-reticuline.

In an embodiment of the invention said modified polypeptide is selected from the group consisting of:
i) a polypeptide comprising a modified amino acid sequence of the amino acid sequence set forth in SEQ ID NO: 9, 10 or 11;
ii) a polypeptide comprising a modified amino acid sequence of the amino acid sequence as set forth in SEQ ID NO: 12, 13 or 14;
iii) a polypeptide comprising a modified amino acid sequence that is at least 95% identical to the full length amino acid sequence as set forth in SEQ ID NO: 9, 10, 11, 12, 13 or 14, wherein said sequence is modified by addition, deletion or substitution of one or more amino acid sequences and wherein said polypeptide has reduced or abrogated enzyme activity in the conversion of (S)-reticuline to 1,2-dehydroreticuline and/or to (*R*)-reticuline when compared to the unmodified polypeptide comprising the amino acid sequence set forth in SEQ ID NO: 9, 10, 11, 12, 13 or 14.

In an embodiment of the invention said modified polypeptide is selected from the group consisting of:
i) a modified polypeptide comprising a modified amino acid sequence of the amino acid sequence set forth in SEQ ID NO: 15;
ii) a modified polypeptide comprising a modified amino acid sequence of the amino acid sequence set forth in SEQ ID NO: 16;
iii) a modified polypeptide comprising a modified amino acid sequence that is at least 95% identical to the full length amino acid sequence as set forth in SEQ ID NO: 15 or 16, wherein said sequence is modified by addition, deletion or substitution of one or more amino acid sequences and wherein said polypeptide has reduced or abrogated enzyme activity in the conversion of 1,2-dehydroreticuline to (*R*)-reticuline when compared to the unmodified polypeptide comprising the amino acid sequence set forth in SEQ ID NO: 15 or 16.

In an embodiment of the invention said modified polypeptide comprises a deletion or substitution of amino acid residue 431 of SEQ ID NO: 9 wherein said modified polypeptide is at least 90% identical to the amino acid sequence set forth in SEQ ID NO: 9.

In an embodiment of the invention said modified polypeptide comprises a deletion or substitution of amino acid residue 432 of SEQ ID NO: 10 wherein said modified polypeptide is at least 90% identical to the amino acid sequence set forth in SEQ ID NO: 10.

In an embodiment of the invention said modified polypeptide comprises a deletion or substitution of amino acid residue 431 of SEQ ID NO: 11 wherein said modified polypeptide is at least 90% identical to the amino acid sequence set forth in SEQ ID NO:11.

In an embodiment of the invention said modified polypeptide is 91%, 92%, 93%, 94%, 95%, 96%, 97%, 98% or 99% identical to the amino acid sequence set forth in SEQ ID NO: 9 wherein said modified polypeptide comprises a deletion or substitution of amino acid residue 431 of SEQ ID NO: 9.

In an embodiment of the invention said modified polypeptide is 91%, 92%, 93%, 94%, 95%, 96%, 97%, 98% or 99% identical to the amino acid sequence set forth in SEQ ID NO: 10 wherein said modified polypeptide comprises a deletion or substitution of amino acid residue 432 of SEQ ID NO: 10.

In an embodiment of the invention said modified polypeptide is 91%, 92%, 93%, 94%, 95%, 96%, 97%, 98% or 99% identical to the amino acid sequence set forth in SEQ ID NO: 11 wherein said modified polypeptide comprises a deletion or substitution of amino acid residue 431 of SEQ ID NO: 11.

In an embodiment of the invention said modified polypeptide comprises a substitution or deletion at amino acid arginine 431 of the amino acid sequence set forth in SEQ ID NO: 9.

In an embodiment of the invention said modified polypeptide comprises a substitution or deletion at amino acid arginine 432 of the amino acid sequence set forth in SEQ ID NO: 10.

In an embodiment of the invention said modified polypeptide comprises a substitution or deletion at amino acid arginine 431 of the amino acid sequence set forth in SEQ ID NO: 11.

In an embodiment of the invention said modification is the substitution of arginine for tryptophan in at position 431 of the amino acid sequences set forth in SEQ ID NO: 9.

In an embodiment of the invention said modification is the substitution of arginine for tryptophan in at position 432 of the amino acid sequences set forth in SEQ ID NO: 10.

In an embodiment of the invention said modification is the substitution of arginine for tryptophan in at position 431 of the amino acid sequences set forth in SEQ ID NO: 11.

In an embodiment of the invention said modified polypeptide comprises the amino acid sequence set forth in SEQ ID NO: 27.

In an embodiment of the invention said modified polypeptide comprises the amino acid sequence set forth in SEQ ID NO: 28.

In an embodiment of the invention said modified polypeptide comprises the amino acid sequence set forth in SEQ ID NO: 29.

In an embodiment of the invention said modified polypeptide is encoded by a nucleic acid molecule comprising a nucleotide sequence set forth in SEQ ID NO: 30.

In an embodiment of the invention said modified polypeptide is encoded by a nucleic acid molecule comprising a nucleotide sequence set forth in SEQ ID NO: 31.

In an embodiment of the invention said modified polypeptide is encoded by a nucleic acid molecule comprising a nucleotide sequence set forth in SEQ ID NO: 32.

Mutagenesis as a means to induce phenotypic changes in organisms is well known in the art and includes but is not limited to the use of mutagenic agents such as chemical mutagens (e.g. base analogues, deaminating agents, DNA intercalating agents, alkylating agents, transposons, bromine, sodium azide] and physical mutagens [e.g. ionizing radiation, psoralen exposure combined with UV irradiation). It is also common general knowledge that ablation of gene function can be obtained through genetic means such as siRNA and antisense. The introduction of double stranded RNA, also referred to as small inhibitory/interfering RNA (siRNA) or short hairpin RNA [shRNA], into a cell which results in the destruction of mRNA complementary to the sequence included in the siRNA/shRNA molecule. This is typically via transfection of expression constructs, for example those disclosed in PCT/GB2015/051446, which are stably integrated into the genome and inherited in a Mendelian manner.

In an embodiment of the invention said modified *Papaver* plant further comprises one or more genes comprising a nucleotide sequence selected from the group consisting of:
i) a nucleotide sequence as represented by the sequence in SEQ ID NO: 17,18, 19, 20, 21, 22, 23, 24, 25 or 26;
ii) a nucleotide sequence wherein said sequence is degenerate as a result of the genetic code to the nucleotide sequence defined in (i);
iii) a nucleic acid molecule the complementary strand of which hybridizes under stringent hybridization conditions to the sequence in SEQ ID NO: 17,18, 19, 20, 21, 22, 23, 24, 25 or 26 wherein said nucleic acid molecule encodes polypeptides involved in the biosynthesis of *Papaver* noscapine or intermediates in the biosynthesis of noscapine.

In an embodiment of the invention said nucleic acid molecule is at least 55%, 60%, 65%, 70%, 75%, 80%, 90%, 91%, 92%, 93%, 94%, 95%, 96%, 97%, 98% or 99% identical to the nucleotide sequence set forth in SEQ ID NO: 17,18, 19, 20, 21, 22, 23, 24, 25 or 26 over the full nucleotide sequence.

In an embodiment of the invention said plant comprises enhanced levels of noscapine or intermediates in the synthesis of noscapine.

In an embodiment of the invention noscapine comprises at least 50% of the benzylisoquinoline alkaloid content of said modified plant.

In a further embodiment of the invention said plant comprises a noscapine content that is at least 55%, 60%, 65%, 70%, 75%, 80%, 85%, 90%, 95%, 96%, 97%, 98%, or 99% of the benzylisoquinoline alkaloid content of said modified plant.

In an alternative embodiment of the invention said plant comprises a noscapine content that is at least 50% of the alkaloid content of a combination of alkaloids comprising morphine, codeine, oriparvine, thebaine and noscapine.

In a further embodiment of the invention said plant comprises a noscapine content that is at least 55%, 60%, 65%, 70%, 75%, 80%, 85%, 90%, 95%, 96%, 97%, 98%, or 99% of the alkaloid content of a combination of alkaloids comprising morphine, codeine, oriparvine, thebaine and noscapine.

In an embodiment of the invention said plant comprises a benzylisoquinoline content that comprises substantially noscapine.

In an embodiment of the invention said Papaver plant is *Papaver somniferum.*

According to a further aspect of the invention there is provided a Papaver capsule obtained from the modified plant according to the invention.

According to a further aspect of the invention there is provided a Papaver straw obtained from the modified plant according to the invention.

According to an aspect of the invention there is provided a Papaver seed obtained from the modified plant according to the invention.

Throughout the description and claims of this specification, the words "comprise" and "contain" and variations of the words, for example "comprising" and "comprises", means "including but not limited to", and is not intended to (and does not) exclude other moieties, additives, components, integers or steps. "Consisting essentially" means having the essential integers but including integers which do not materially affect the function of the essential integers.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

### Definitions

"Benzylisoquinoline alkaloids" typically include papaverine, noscapine, codeine, morphine, apomorphine, berberine, protopine, tubocurarine and sanguinarine. The major alkaloids are morphine, codeine, oriparvine, thebaine and noscapine.

"Substantially noscapine" means that other benzylisoquinoline alkaloids are trace alkaloids or undetectable benzylisoquinoline alkaloids as measured by techniques available to the skilled person.

"Stably inherited" refers to the transfer of relevant characteristics which remain unchanged after repeated propagation or, in the case of a particular cycle of propagation, at the end of each such cycle.

"Reduced or abrogated expression" refers to reduction of mRNA expression by 10, 20, 30, 40, 50, 60, 70, 80, 90 or 100% when compared to a wild-type plant expressing a gene encoding an unmodified cytochrome p450-oxidoreductase. Quantitative mRNA expression methods are known to the skilled person such for example real time PCR.

"Functional cytochrome P450-oxidoreductase" refers to a non-modified enzyme which is capable of converting (S)- to (R)- reticuline via 1,2-dehydroreticuline. The measurement of cytochrome P450 and oxidoreductase activities are well known in the art, for example see Lenz & Zenk, 1995, FEBS 233:132-139. In addition heterologous expression of cytochrome P450 and oxidoreductase domains in addition to expression of cytochrome P450-oxidoreductase fusion protein in microbial systems such as yeast allows functional characterisation of functional and non-functional cytochrome P450-oxidoreductase enzymes.

The phrase "enhanced levels of noscapine and noscapine intermediates" means producing a higher level of noscapine or intermediates in the synthesis of noscapine compared to a wild-type plant comprising a functional cytochrome P450-oxidoreductase, for example, a 10%, 20%, 30%, 40%, 50%, 60%, 70%, 80%, 90%, 95%, 96%, 97%, 98% or 99% increase in the level of noscapine or intermediates in noscapine biosynthesis.

"Wild-type plant" means a plant having a phenotype that is characteristic of the species in a natural breeding population.

"Reduced or abrogated activity in the conversion of (*S*)-reticuline to (*R*)-reticuline" refers to the reduction of the catalytic activity of the enzyme by 10, 20, 30, 40, 50, 60, 70, 80, 90 or 100% relative to an unmodified cytochrome P450-oxidoreductase.

Variant as referred to under "SEQ ID Summary" refers to different polymorphic forms of the particular gene.
SEQ ID NO: 1 P450 domain nucleotide sequence; polymorphic variant a
SEQ ID NO: 2 P450 domain nucleotide sequence; polymorphic variant b
SEQ ID NO: 3 P450 domain nucleotide sequence; polymorphic variant c
SEQ ID NO: 4 oxidoreductase A nucleotide sequence, variant b
SEQ ID NO: 5 oxidoreductase B nucleotide sequence
SEQ ID NO: 6 cytochrome P450-oxidoreductase nucleotide sequence; polymorphic variant a
SEQ ID NO: 7 cytochrome P450-oxidoreductase nucleotide sequence; polymorphic variant b
SEQ ID NO: 8 cytochrome P450-oxidoreductase nucleotide sequence; polymorphic variant c
SEQ ID NO: 9 cytochrome P450-oxidoreductase amino acid, polymorphic variant a
SEQ ID NO: 10 cytochrome P450-oxidoreductase amino acid; polymorphic variant b
SEQ ID NO: 11 cytochrome P450-oxidoreductase amino acid; polymorphic variant c
SEQ ID NO: 12 P450 domain amino acid; polymorphic variant a
SEQ ID NO: 13 P450 domain amino acid; polymorphic variant b
SEQ ID NO: 14 P450 domain amino acid; polymorphic variant c
SEQ ID NO: 15 oxidoreductase A amino acid
SEQ ID NO: 16 oxidoreductase B amino acid
SEQ ID NO: 17 PSMT1
SEQ ID NO: 18 PSMT2
SEQ ID NO: 19 PSMT3
SEQ ID NO: 20 CYP450 1 (CYP 82X1)
SEQ ID NO: 21 CYP450 2 (CYP 719A21)
SEQ ID NO: 22 CYP450 3 (CYP 82X2)
SEQ ID NO: 23 CYP450 4 (CYP 82Y1)
SEQ ID NO: 24 PSAT1
SEQ ID NO: 25 PSSDR1
SEQ ID NO: 26 PSCXE1
SEQ ID NO: 27 cytochrome P450-oxidoreductase mutant amino acid, variant a
SEQ ID NO: 28 cytochrome P450-oxidoreductase mutant amino acid, variant b
SEQ ID NO: 29 cytochrome P450-oxidoreductase mutant amino acid, variant c
SEQ ID NO: 30 cytochrome P450-oxidoreductase mutant nucleotide sequence variant a
SEQ ID NO: 31 cytochrome P450-oxidoreductase mutant nucleotide sequence variant b
SEQ ID NO: 32 cytochrome P450-oxidoreductase mutant nucleotide sequence variant c
SEQ ID NO: 33 Oxidoreductase A, nucleic acid sequence, variant a (and c)

An embodiment of the invention will now be described by example only and with reference to the figures, examples and tables:

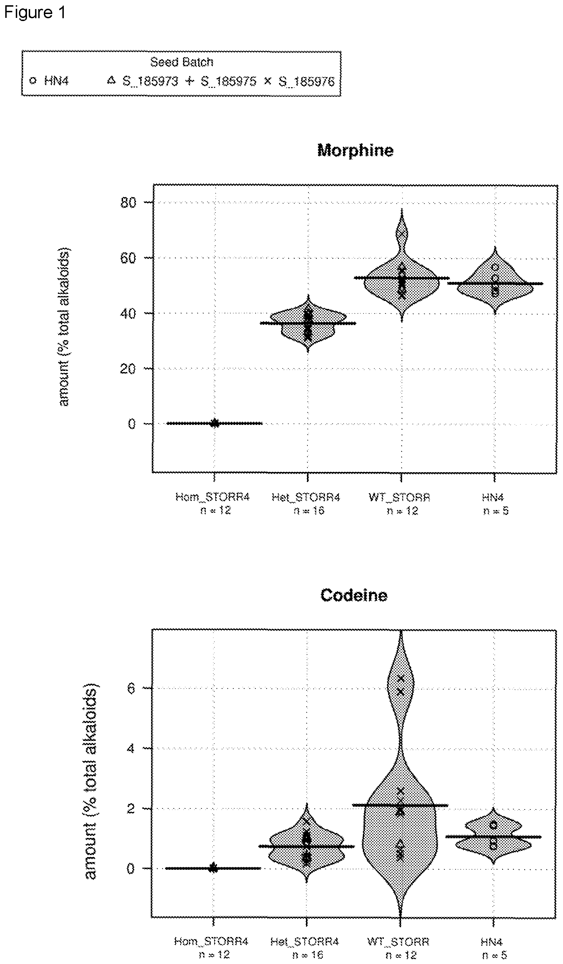

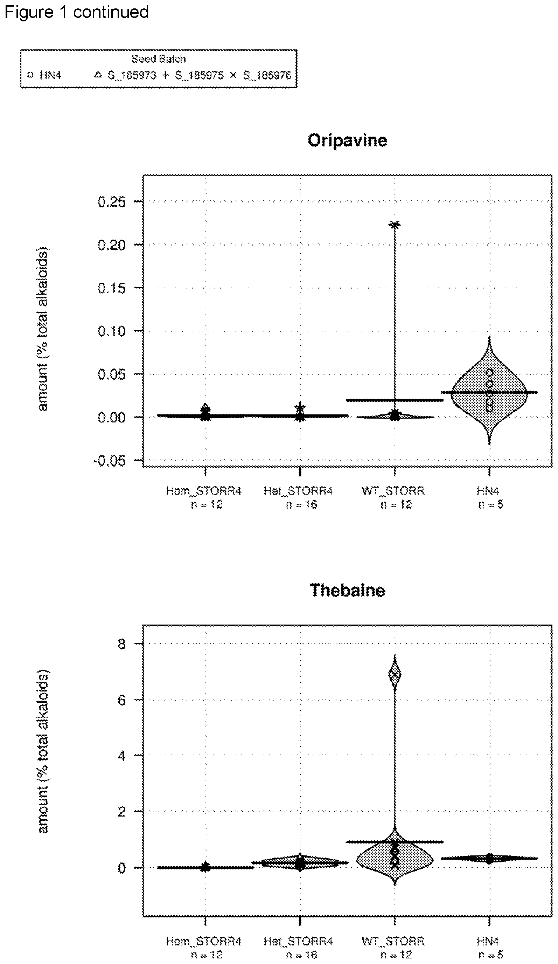

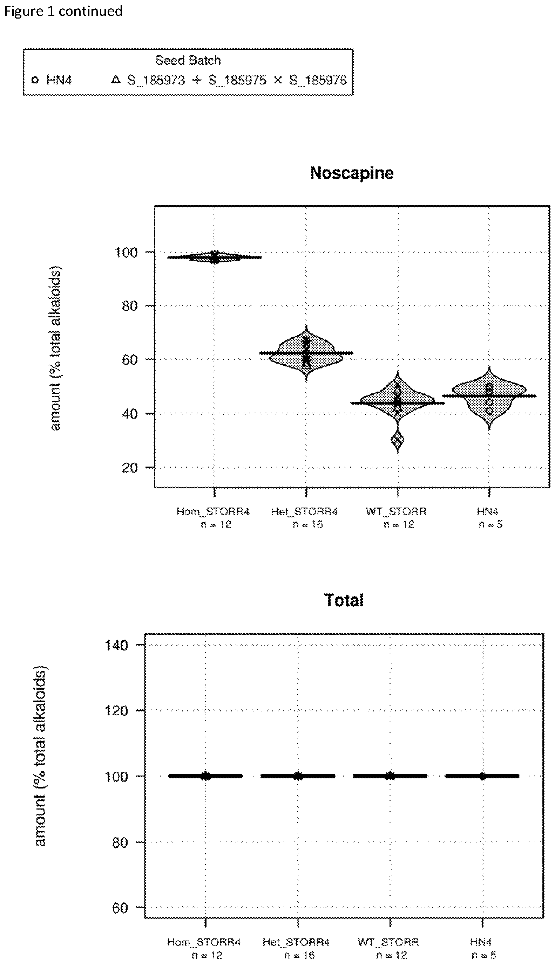

Figure 1: Violin plot of the relative amount (% total) of noscapine and morphinan alkaloids in dried capsules of F2 populations segregating *storr-4.* HOM_STORR4, F2 material homozygous for *storr-4;* HET_STORR4, F2 material heterozygous for *storr-4;* WT_STORR, F2 material homozygous for the STORR wild type allele; HN4, control plants of NOSCAPINE CVS 4, one of the parental lines of the F2 material;

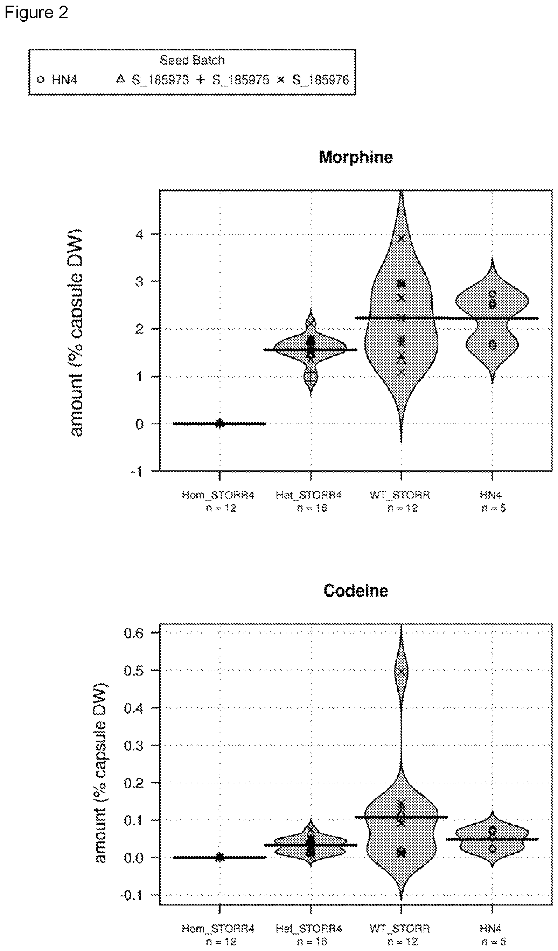

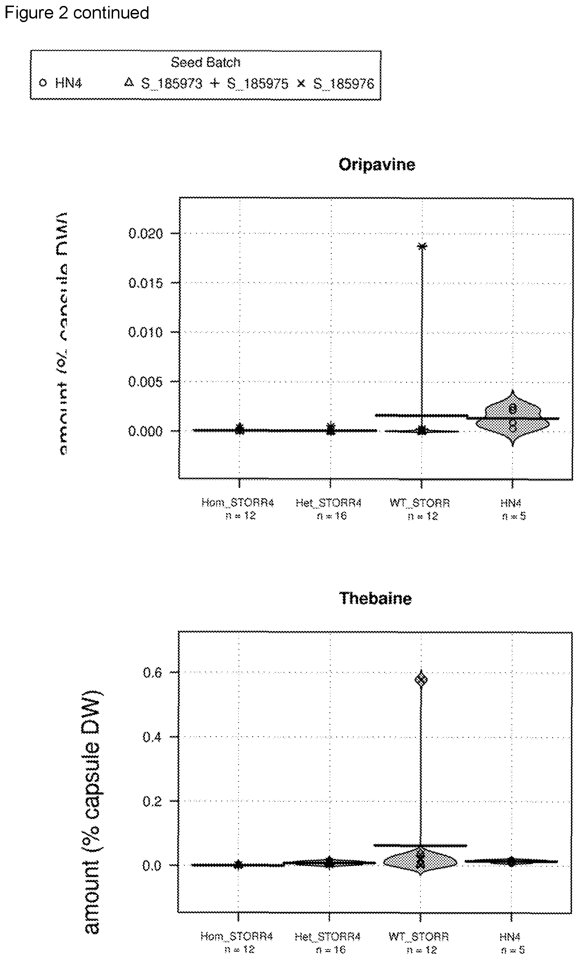

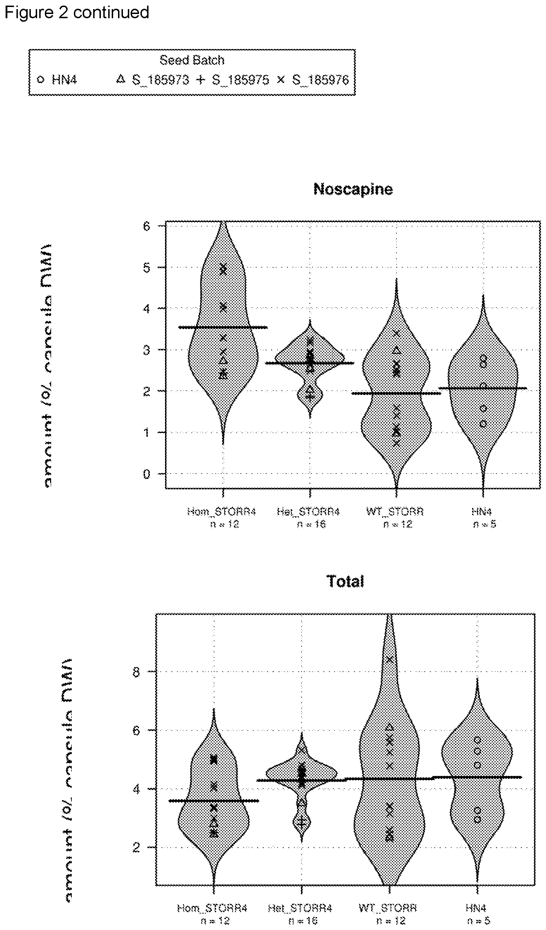

Figure 2: Violin plot of the absolute amount (% capsule dry weight) of noscapine and morphinan alkaloids in dried capsules of segregating F2 populations segregating *storr-4.* HOM_STORR4, F2 material homozygous for *storr-4;* HET_STORR4, F2 material heterozygous for *storr-4;* WT_STORR, F2 material homozygous for the STORR wild type allele; HN4, control plants of NOSCAPINE CVS 4, one of the parental lines of the F2 material; DW, dry weight; and

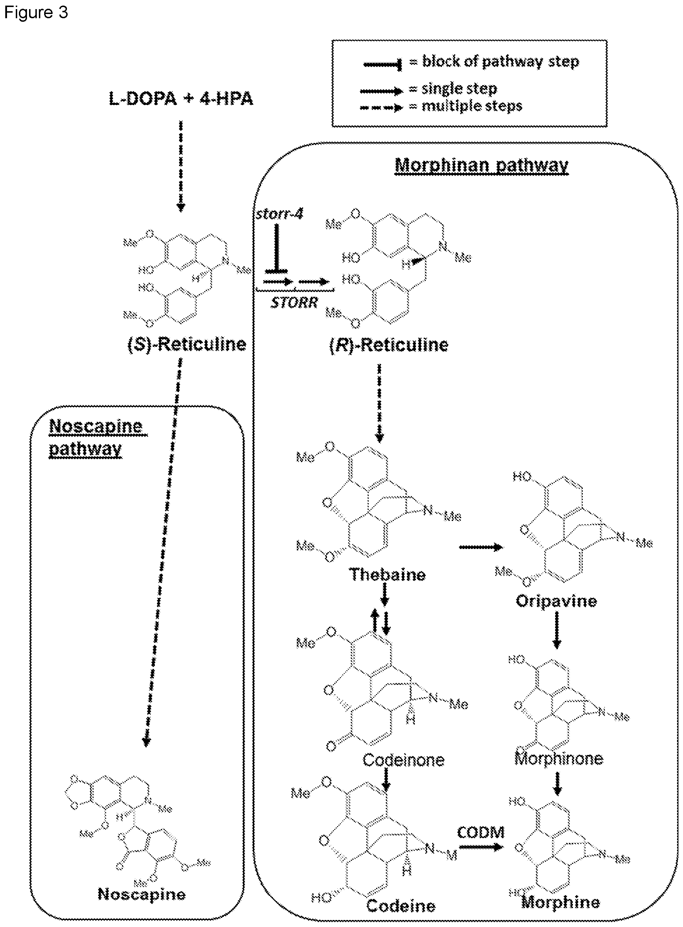

Figure 3: Schematic overview of noscapine and morphinan biosynthetic pathways in opium poppy and the position of the pathway block conferred by *storr-4.* (*S*)-reticuline is a major branch point intermediate in alkaloid metabolism in opium poppy serving as a precursor for both noscapine and morphinan biosynthesis. The *STORR* locus controls the gateway reaction into morphinan biosynthesis, the two step epimerisation of (*S*)- to (*R*)-reticuline. Impairment of STORR, for example by means of knock out mutations such as *storr-4,* leads to a block of morphinan biosynthesis and increased production of noscapine in noscapine producing cultivars of opium poppy.

### Materials & Methods

### EMS mutagenesis

Prior to mutagenesis, M0 seeds from *P. somniferum* cultivar GSK NOSCAPINE CVS2 were soaked in 0.1% (w/v) potassium chloride solution for approximately 24 hours. The seed was then imbibed for three hours with ethyl methanesulfonate (EMS) solution (200 mM EMS in 100 mM sodium hydrogen phosphate buffer, pH 5.0, supplemented with 700 mM dimethyl sulfoxide). The treated M1seed was washed twice for 15 minutes each with 100 mM sodium thiosulphate solution and then twice for 15 minutes each with distilled water. A volume of 10 mL of the respective solutions was used per 1000 seeds and all steps were carried out on a rocking platform. The washed M1 seed was dried overnight on Whatman 3MM Blotting paper (Whatman/GE Healthcare Life Sciences, Little Chalfont, UK) and sown the following morning on top of a mixture of John Innes no 2 compost, vermiculite and perlite (4:2:1), then covered with a thin layer of compost. Seedlings were transplanted three to four weeks after germination into larger pots and grown in the glasshouse until maturity. Dried capsules were harvested by hand from the M1 population once capsules had dried to approximately 10% moisture on the plant. M2 seed was manually separated from the capsule, providing the M2 seed families that were used in the field-based trials described below.

### Field-based trials of EMS-mutagenised lines

M2 seed was sown in in the field in Tasmania in the 2009/2010 growing season. M2 seed lines from the EMS mutagenised population were hand sown in 3m long rows. After germination, plants were thinned to 75 mm apart. Typically 4 plants (designated A, B, C, D) per M2 line were self-pollinated (two immature buds per plant selected) by securing a linen bag over the bud. Open-pollinated rows were harvested at ~ 11% moisture, and assayed (as described below) for morphine (M), codeine (C), oripavine (O) and thebaine (T) content. Analysis of a selected self-pollinated capsules from an M2 plants indicated significantly reduced morphinan assays, and significantly higher assays of noscapine. M3 and M4 seeds derived from a selected self-pollinated M2 capsule displaying the low morphinan alkaloids/high noscapine phenotype were sown in the following growing seasons in Tasmania to confirm this phenotype in both open-pollinated and self-pollinated capsules. Typically 4 plants (designated A, B, C, D) were self-pollinated per M3 and M4 seed line as described above. GSK commercial cultivars were grown alongside the M2, M3 and M4 lines as a comparison. Depending on the season, they included GSK MORPHINE CULTIVARs 3 and 5, GSK THEBAINE CULTIVAR 3, GSK NOSCAPINE CULTIVARs 3 - 6.

### Poppy straw analysis of field-based trials

Poppy capsules were harvested from the respective mutant lines by hand once capsules had dried to approximately 10% moisture on the plant. The seed was manually separated from the capsule, and capsule straw material (poppy straw) was then shipped to the GSK extraction facility in Port Fairy, Australia.

The poppy straw samples were ground in a Retsch Model MM400 ball mill into a fine powder. Two gram samples of ground poppy straw (2 ± 0.003 g) were extracted in 50 mL of a 10% acetic acid solution. The extraction suspension was shaken on an orbital shaker at 200rpm for a minimum of 10 minutes then filtered to provide a clear filtrate. The final filtrate was passed through a 0.22 µm filter prior to analysis.

The solutions were analysed using a Waters Acquity UPLC system (Waters Corporation, Milford, MA, USA) fitted with a Waters Acquity BEH C18 column, 2.1mm × 100mm with 1.7 micron packing. The mobile phase used a gradient profile with eluent A consisting of 0.1% Trifluoroacetic acid in deionised water and eluent B consisting of 100% Acetonitrile. The mobile phase gradient conditions used are as listed in Table 1, the gradient curve number as determined using a Waters Empower chromatography software package. The flow rate was 0.6 mL per minute and the column maintained at 45 degrees centigrade. The injection volume was 1 µL injection volume and the alkaloids were detected using a UV detector at 285 nm.

The loss on drying (LOD) of the straw was determined by drying in an oven at 105 degrees centigrade for 16-20 hours.

**Table 1 - Gradient Flow Program**

| **TIME (minutes)** | **% Eluent A** | **% Eluent B** | **Flow (mL/min)** | **Curve No** |
|---|---|---|---|---|
| 0.00 | 95.0 | 5.0 | 0.60 | INITIAL |
| 0.80 | 90.0 | 10.0 | 0.60 | 6 |
| 3.40 | 75.0 | 25.0 | 0.60 | 3 |
| 3.60 | 95.0 | 5.0 | 0.60 | 6 |
| 4.00 | 95.0 | 5.0 | 0.60 | 11 |

Alkaloid concentrations for morphine, pseudomorphine, codeine, thebaine, oripavine and noscapine were quantified using external standard calibration and the results calculated on a dry weight basis. Reticuline was quantified using the thebaine calibration (assuming the thebaine response) and results calculated on a dry weight basis (% weight relative to thebaine; %WRT).

Typical retention times are as follows:

| **Compound** | **Retention Time (minutes)** |
|---|---|
| Morphine | 1.14 |
| Pseudomorphine | 1.26 |
| Codeine | 1.69 |
| Oripavine | 1.80 |
| Reticuline | 2.05 |
| 10-Hydroxythebaine | 2.32 |
| Thebaine | 2.53 |
| Noscapine | 3.16 |

**Leaf latex analysis of glasshouse grown M4 generation (S-186668) derived from the self-pollinated MCS 746 C plant.**

The latex alkaloid profile determined in M4 siblings of the plants used for sequencing the cytochrome P450-oxidoreductase (*STORR*) cDNA (see below). Latex was collected when the first flower buds emerged (~7 week old plants) from cut petioles, with a single drop dispersed into 500 µL of 10% acetic acid. This was diluted 10× in 1% acetic acid to give an alkaloid solution in 2% acetic acid for further analysis. The samples were analysed by UPLC-MS and R-scripts as previously described ("the standard method"; (Winzer T. et al. (2012) Science 336, 1704-8)). Compounds were annotated by generation of empirical formulae from exact masses (< 5 ppm mass accuracy) and comparison to authentic standards. Unknowns were annotated by a masstag in the format MxTy, where x is mass and y is retention time in seconds.

The relative content of alkaloids was measured as % peak area relative to total alkaloid peak area.

**Isolation and sequencing the of the cytochrome P450-oxidoreductase cDNA in the M4 generation (S-186668) derived from the self-pollinated MCS 746 C plant.**

RNA was isolated from M4 individuals derived from self-pollinated M3 individual MCS 746 C that displayed the low morphinan alkaloid/high noscapine phenotype. M4 plants were grown under glasshouse conditions to post-flowering stages (1-6 days after petal fall) and stem segments (2.5-3 cm long) immediately beneath the flowers were harvested and flash frozen in liquid nitrogen.

Total RNA was extracted from the stems using the pine tree RNA extraction method (Chang et al., 1993. Plant Mol. Biol. Rep. 11: 113-116) and further purified using the RNeasy Plus MicroKit (Qiagen, Crawley, UK).

Synthesis of cDNA was performed using 1 µg of total RNA, 10 µM oligo dT MW 4500 (Invitrogen/Life technologies, Paisley, UK) and 1 mM dNTP. Reactions were incubated for 5 min at 65°C to allow annealing of oligonucleotides. First strand synthesis reactions contained 1 X First Strand buffer (Invitrogen/Life Technologies), 20 mM DTT, 40 U RNAse out (Invitrogen/Life technologies). Reactions were incubated at 42°C for 2 min and then 200 U SuperScript II reverse transcriptase (Invitrogen/Life technologies, Paisley, UK) was added followed by a 50 min incubation at 42°C and heat inactivation at 70°C for 15 min. Samples were diluted 5 X in water and used for gene specific amplifications.

The full-length region encoding the cytochrome P450-oxidoreductase gene was amplified from the cDNA samples using the primers 5'- ATGGAGCTCCAATATATTTC-3', 5'-TCAAGCTTCATCATCCCACA-3' and the high fidelity Q5^{®} Hot Start High-Fidelity DNA Polymerase (New England Biolabs, Hitchin, UK) according to the guidelines supplied by the manufacturer. Cycling conditions were as follows: 98°C for 30 s, 30 cycles of 98°C for 10 s, 60°C for 30 s, 72°C for 1.5 min and a final extension at 72°C for 2 min. The 3 kb-PCR product was then ligated into the pJET1.2 cloning vector using the CloneJET PCR Cloning Kit (Thermo Scientific) according to the manufacturer's protocol. Three individual clones were fully sequenced with the sequencing primer shown in the Table 1.

**Table 1: Sequencing primers used to sequence the cytochrome P450-oxidoreductase cDNA M4 progeny of MCS 746 C.**

| SEQ ID NO | Oligonucleotides sequence (5' to 3') |
|---|---|
| pJET1.2 Forward Sequencing primer (SEQ ID NO 37) | CGACTCACTATAGGGAGAGCGGC |
| pJET1.2 Reverse Sequencing primer (SEQ ID NO 38) | AAGAACATCGATTTTCCATGGCAG |
| Primer 1 (SEQ ID NO 39) | GCAGTGCTTCATATTTCTCG |
| Primer 2 (SEQ ID NO 40) | CCTTATGGAAAATATTGGAGGGAGC |
| Primer 3 (SEQ ID NO 41) | GTCTCTCTCAGAGGCTGCT |
| Primer 4(SEQ ID NO 42) | TTGTCTACATCCAGGCAATC |

### RNA-seq of stems and capsules from GSK NOSCAPINE CVS 3.

Stem and capsule material was harvested from stems and capsules of GSK NOSCAPINE CVS 3 plants 1-2 days after petal fall. RNA was prepared individually from five plants. The harvested material was ground in liquid nitrogen using a mortar and pestle. RNA was isolated from the ground stem or capsule preparations as previously described (Chang et al., 1993, Plant Mol. Biol. Rep. 11: 113-116) with slight modifications (three extractions with chloroform:isoamylalcohol, RNA precipitation with Lithium chloride at -20°C overnight). RNA was quantified spectrophotometrically before pooling equal amounts of RNA from five plants per cultivar, stage and organ. The pooled samples underwent a final purification step using an RNeasy Plus MicroKit (Qiagen, Crawley, UK) to remove any remaining genomic DNA from the preparations. RNA was eluted in 100 µL RNase-free water.

RNA sequencing was performed on one lane of the Illumina HiSeq 2000 platform (Illumina, Little Chesterford, UK) using the TruSeq RNA kit. TruSeq RNA library construction was carried out according to the manufacturer's description and included a poly-A selection step.

De novo sequence assembly of the raw sequence datasets was performed using the Trinity RNA-seq de novo assembly tool (Haas et al., 2013, Nature Protocols, 8: 1494-1512; http://trinityrnaseq.github.io/).

### Mutation discovery in the STORR locus

The clonal STORR cDNA sequences obtained from M4 progeny of plant MCS 746 C displaying the low morphinan alkaloid/high noscapine phenotype were compared to the corresponding contiguous sequence assembled from RNA-seq data from GSK NOSCAPINE CVS 3, the opium poppy cultivar used for EMA mutagenesis. The clonal and contiguous sequences were identical apart from a C to T transitions at position 1291 with respect to SEQ ID NO: 6. This C to T transition is consistent with the type of mutations introduced by EMS (Greene et al., 2003, Genetics, 164: 731-740). The mutation results in a arginine to tryptophan amino acid substitution at position 431 of the STORR protein (R431W).

### Enzyme assays

General reaction set up was carried out as previously described (Lenz & Zenk, 1995, FEBS 233:132-139). Substrates (*S*)-reticuline, 1,2-dehydroreticuline and coclaurine were purchased from TRC Chemicals (Toronto, Canada) and codeinone was supplied by MacFarlan-Smith (Edinburgh, UK). Assay reactions contained 300 mM NADPH, 5 µg oxidoreductase A or B preparation, 100 mM buffer covering a range of pH values (potassium phosphate pH 6-8, glycine-NaOH pH 9) and 75 µM substrate. Reactions were incubated for 2 h at 37°C and immediately frozen at -80°C. Reactions were dried down to powder in speedvac Gene Vac EZ-2 plus (Ipswich, UK) at 40°C and resuspended in 100 µL 1:1 Hexane: Ethanol (v/v), 0.1% Diethylamine (v/v).

Alternatively enzyme assays were set up in 250 µL volumes containing final concentrations of 50 mM Bis-tris propane buffer, 1 mM NADPH and 100 µM of substrate prepared from transformed yeast recombinantly expressing wild-type and mutant cytochrome P450-oxidoreductases. The assays were started by the addition of 50 µL of 2 mg mL⁻¹ soluble or microsomal extract from transformed yeast, and incubated at 37 °C for 3 hours. After this time, 50 µL of the extract was removed and quenched by the additional of 50 µL of methanol + 1 % acetic acid containing 100 mM noscapine as an internal standard. The reaction was then centrifuged at 20,000 × *g* for 2 min, and the supernatant recovered for analysis of 1,2-dehydroreticuline and (*R*/*S*) -reticuline content (i.e., non-chiral method). The remaining 200 µL of assay was used for chiral analysis. The reaction was quenched by the addition of 0.5 M sodium carbonated buffer (pH 10.0). Reticuline was then extracted from the reaction products by three sequential extractions with 400 µL of dichloromethane. The solvent extracts were combined and evaporated to dryness in a speedvac. The extract was then dissolved in 100 µL of 50:50:0.1 hexane/ethanol/diethylamine and analysed by the chiral HPLC method to determine the relative amounts of (*R*)- and (*S*)-reticuline.

**EMS mutagenesis of a noscapine opium poppy cultivars leads to opium poppy lines where noscapine constitutes more than 90% of the total alkaloid content.**

Among the plants derived from M2 seed line S-121435 one individual - designated plant A - displayed a high noscapine and low morphinan alkaloid phenotype compared to noscapine, thebaine and morphine cultivars (Table 3). The relative noscapine content expressed as percentage of total major alkaloids (morphine, codeine, oripavine, thebaine and noscapine) reached almost 85% in this plant. The relative content of noscapine further increased and that of morphinan alkaloids further decreased in the M3 and M4 progeny both in self-and open-pollinated capsules indicating that the underlying mutation is no longer segregating in these populations. Likewise, noscapine amounts to more than 90% of the relative alkaloid content in the latex of glasshouse-grown M4 siblings of plants used for sequencing the cytochrome P450-oxidoreductase (STORR) cDNA (Table 2). This shows that the mutant phenotype is stable in the M4 generation and across environmental and developmental conditions.

### Generation of F2 populations population segregating storr-4 in the background of high noscapine cvs 4 (HN4)

The impact of the *storr-4* allele on noscapine and morphinan alkaloid accumulation was assessed in segregating F2 populations. M4 mutant plants derived from seed batch S-186668 were crossed with high NOSCAPINE CVS4 (HN4). The resulting F1 generation was self-pollinated to produce F2 seed lines segregating storr-4 in a noscapine background. Three F2 lines (S-185973, S-185975, S-185976) were grown along HN4 control plants in the glasshouse to maturity. Dried capsule material was harvested and analysed as described below.

### Genotyping of storr-4

KASP genotyping assays for *storr-4* were designed to genotype F2 populations. To design allele-specific primers, sequences of 50-100 nucleotides around the mutation 10 site were submitted to LGC genomics (Hoddesdon, UK) to order KASP by Design (KBD) primer mix. The sequences of the allele-specific primers as well as the common primer are shown in Table 4.

**Table 4. Primer sequences, in 5'-3' orientation.**

| **Mutant allele** | **Primer** | **Primer sequence** | **Label** |
|---|---|---|---|
| *storr-4* | Wild type allele (SEQ ID NO 34) | CCAGGCAATCATCAAAGAATCAATGC | FAM |
| | Mutant allele (SEQ ID NO 35) | CCAGGCAATCATCAAAGAATCAATGT | HEX |
| | Common primer (SEQ ID NO 36) | CACGGGGCTGGCTGGATACAA | |

Leaf samples (30-50mg) for DNA extraction were harvested from 4-6 week old plants. Genomic DNA was extracted using the BioSprint 96 Plant kit on the BioSprint 96 Workstation (Qiagen, Crawley, UK) according to the manufacturer's protocol. Extracted DNA was quantified on the NanoDrop^{™} 8000 Spectrophotometer (Fisher Scientific, Wilmington, DE, USA) and normalized to 5 ng/uL.

KASP assay reactions were carried out in FrameStar^{®} 384 well plates (4titude^{®} Ltd, Wotton, UK) in a 5.07 µL volume per reaction, with the following reaction components: 2x KASP V4.0 mastermix (LGC genomics), 2.5 µL; KBD primer mix, 0.07 µL; HyClone molecular grade water (Thermo Scientific, Hemel Hempstead, UK), 0.5 µL; 5 ng/uL DNA, 2 µL. The plates were centrifuged for 2 minutes at 4500 × *g*, heat-sealed using the Kube^{™} heat-based plate sealer (LGC genomics), and thermal cycling was carried out in a Hydrocycler^{™} (LGC genomics) water bath-based thermal cycler, with the following conditions: 94°C for 15 minutes; 10 cycles: 94°C for 20 seconds, 61-55°C for 60 seconds (dropping 0.6°C per cycle); 34 cycles: 94°C for 20 seconds, 55°C for 60 seconds. The plate reading was carried out on an Applied Biosystems ViiA7 instrument (Life Technologies), for 30 seconds at 25°C.

### Capsule straw analysis of glasshouse grown F2 populations

Capsules from the F2 populations and HN4 controlswere harvested once capsules had dried to approximately 10% moisture on the plants. Seed was manually separated from the capsule and single capsules were ground to a fine powder in a ball mill (Model MM04, Retsch, Haan, Germany). Samples of ground poppy straw were then weighed accurately to 10 ± 0.1 mg and extracted in 0.5 mL of a 10% (v/v) acetic acid solution with gentle shaking for 1h at room temperature. Samples were then clarified by centrifugation and a 50 µL subsample diluted 10× in 1% (v/v) acetic acid to give an alkaloid solution in 2% acetic acid for further analysis. All solutions were analysed as described for the poppy straw analysis from field grown plants. Likewise, all data analysis was carried out using the R programming language. Putative alkaloid peaks were quantified by their pseudomolecular ion areas using custom scripts. Alkaloids were identified by comparing exact mass and retention time values to those of standards. Where standards were not available, the Bioconductor rcdk package (Smith et al., 2006), Anal. Chem. 78 (3): 779-787) was used to generate pseudomolecular formulae from exact masses within elemental constraints C = 1 100, H = 1 200, O = 0 200, N = 0 3 and mass accuracy < 5ppm. The hit with the lowest ppm error within these constraints was used to assign a putative formula.

### Exam ples

**The *storr-4* allele co-segregates with the high noscapine zero morphinan phenotype.** F2 populations segregating *storr-4* in a high noscapine background were genotyped for *storr-4* and their relative and absolute alkaloid content determined in dried capsules. The *storr-4* mutant allele strictly co-segregated with extremely high noscapine and zero morphinan levels (Figure 1 and 2). The relative noscapine content in homozygous *storr-4* material reached 98 ± 0.7 % with the remainder being mainly reticuline (1.8 ± 0.7 %). Noscapine and morphinan levels of heterozygous F2 material were intermediate between those of homozygous *storr-4* F2 material and F2 plants homozygous for the STORR wild type allele (Figure 1 and 2). The alkaloid profile of F2 plants homozygous for the *STORR* wild type allele was overall very similar to that of control plants from HN4, one of the parental varieties used to generate the F2 population.

The data clearly demonstrate that the shutdown of morphinan biosynthesis in *STORR* knock out mutations such as *storr-4* allows leads to increased metabolic flux into the noscapine branch pathway (Figure 3) thus resulting in extremely high levels of noscapine.

**Table 2**

| Latex data from glasshouse-grown M4 progeny (S-186668) of plant MCS 746 C | | | | | | | |
|---|---|---|---|---|---|---|---|
| **Alkaloid content % total alkaloids** | | | | | | | |
| **Morphine** | **Codeine** | **Codeinone** | **Oripavine** | **Thebaine** | **Noscapine** | **Laudanosin** | **Protopine** |
| 1.3 ± 2.32 | 0.34 ± 0.71 | 0.01 ± 0 | 0 ± 0 | 0.07 ± 0.1 | 92.34 ± 3.35 | 0.81 ± 0.38 | 0.2 ± 0.22 |
| averages and standard deviation of 15 individual M4 plants are shown | | | | | | | |

**Table 3: alkaloid content (% dry weight and % total alkaoind content) in different plant lines**

| **Season** | **Line** | **Generation** | **Self-/open pollinated *** | **Alkaloid content % dry weight Morphine Codeine Oripavine ThebaineNoscapine** | | | | | **Alkaloid content % total alkaloids** Codeine Oripavine ThebaineNoscapine** | | | | |
|---|---|---|---|---|---|---|---|---|---|---|---|---|---|
| 2012/13 | GSK MORPHINE CVS 4 | | OP row | 3.520 | 0.310 | 0.050 | 0.170 | 0.000 | 86.9 | 7.7 | 1.2 | 4.2 | 0.0 |
| | GSK NOSCAPINE CVS 3 | | OP row | 2.630 | 0.180 | 0.015 | 0.030 | 2.550 | 48.7 | 3.3 | 0.3 | 0.6 | 47.2 |
| | S-121435 | M2 | OP row | 1.780 | 0.140 | 0.010 | 0.060 | 2.850 | 36.8 | 2.9 | 0.2 | 1.2 | 58.9 |
| | | | SP cap A | 0.590 | 0.060 | 0.020 | 0.080 | **4.220** | 11.9 | 1.2 | 0.4 | 1.6 | **84.9** |
| 2013/14 | GSK MORPHINE CVS 5 | | OP row | 3.840 | 0.140 | 0.040 | 0.135 | 0.090 | 90.5 | 3.3 | 0.9 | 3.2 | 2.1 |
| | GSKTHEBAINE CVS 3 | | OP row | 0.970 | 0.210 | 1.150 | 2.970 | 0.060 | 18.1 | 3.9 | 21.5 | 55.4 | 1.1 |
| | GSK NOSCAPINE CVS 4 | | OP row | 2.225 | 0.185 | 0.115 | 0.25 | 1.955 | 47.0 | 3.9 | 2.4 | 5.3 | 41.3 |
| | MC 746 (progeny of S-121435 self-pollinated capsue A) | M3 | OP row | 0.27 | 0.06 | 0.01 | 0.07 | **3.370** | 7.1 | 1.6 | 0.3 | 1.9 | **89.2** |
| | | | SP caps A-D | 0.19 ± 0.295 | 0.033 ± 0.013 | 0.01 ± 0 | 0.07 ± 0.046 | **3.47 ± 1.39** | 4.05 ± 4.95 | 1.03 ± 0.74 | 0.31 ± 0.13 | 1.70 ± 0.56 | **92.91 ± 4.87** |
| 2014/15 | GSK NOSCAPINE CVS 3 | | OP row | 2.125 | 0.145 | 0.025 | 0.065 | 1.885 | 50.1 | 3.4 | 0.6 | 1.5 | 44.4 |
| | GSK NOSCAPINE CVS 4 | | OP row | 1.145 | 0.080 | 0.030 | 0.175 | 2.520 | 29.0 | 2.0 | 0.8 | 4.4 | 63.8 |
| | GSK NOSCAPINE CVS 5 | | OP row | 2.325 | 0.095 | 0.01 | 0.035 | 1.995 | 52.1 | 2.1 | 0.2 | 0.8 | 44.7 |
| | GSK NOSCAPINE CVS 6 | | OP row | 2.375 | 0.165 | 0.015 | 0.055 | 1.71 | 55.0 | 3.8 | 0.3 | 1.3 | 39.6 |
| | MD 2717 (progeny of MC 746 self-pollinated capsule A) | M4 | OP row | 0.020 | 0.020 | 0.010 | 0.080 | 3.610 | 0.530 | 0.530 | 0.270 | 2.140 | 96.520 |
| | | | SP caps A-D | 0.028 ± 0.005 | 0.035 ± 0.013 | 0.02 ± 0 | 0.058 ± 0.019 | **4.108 ± 0.39** | 0.655 ± 0.152 | 0.833 ± 0.322 | 0.474 ± 0.046 | 1.377 ± 0.517 | 96.661 ± 0.717 |
| * OP = open pollinated; SP-cap(s) = self-pollinated capsule(s), | | | | | | | | | | | | | |
| ** based on main alkaloids: Morphine, Codeine, Oripavine, Thebaine and Noscapine | | | | | | | | | | | | | |

## Claims

1. A plant of the genus *Papaver* wherein said plant is modified in a gene comprising a nucleotide sequence as set forth in SEQ ID NO: 6, 7 or 8, or a nucleic acid molecule which is at least 90% identical to the nucleotide sequence set forth in SEQ ID NO 6, 7 or 8 over the full nucleotide sequence and encodes a modified cytochrome P450-oxidoreductase catalysing the conversion of (*S*)-reticuline to (*R*)-reticuline wherein said modification is homozygous, stably inherited and reduces or abrogates the expression or activity of said cytochrome P450 domain and/or oxidoreductase domain of said modified polypeptide wherein noscapine comprises at least 50% of the benzylisoquinoline alkaloid content of said modified plant.

2. The plant according to claim 1 wherein said modified nucleic acid molecule is:
i) at least 91%, 92%, 93%, 94%, 95%, 96%, 97%, 98% or 99% identical to the nucleotide sequence set forth in SEQ ID NO: 6, 7 or 8 over the full nucleotide sequence; or
ii) is at least 99% identical to the nucleotide sequence set forth in SEQ ID NO 6, 7 or 8, over the full nucleotide sequence and wherein said modification is an addition, substitution or deletion of at least one nucleotide base between nucleotides 1280-1300 set forth in SEQ ID NO 6, 7 or 8.

3. The plant according to claim 2 wherein said modification is a substitution of at least one nucleotide base between nucleotides 1280-1300 set forth in SEQ ID NO 6, 7 or 8 or wherein said modification is a substitution of at least one nucleotide base between nucleotides 1285-1295 set forth in SEQ ID NO 6, 7 or 8 or wherein said modification is a C to T substitution of the nucleotide base at nucleotide 1291 of SEQ ID NO 6, 7 or 8.

4. The plant according to any one of claims 1 to 3 wherein said modified polypeptide has reduced or abrogated activity and is selected from the group consisting of:
i) a polypeptide comprising a modified amino acid sequence of the amino acid sequence set forth in SEQ ID NO: 9, 10 or 11;
ii) a polypeptide comprising a modified amino acid sequence that is at least 95% identical to the full length amino acid sequence as set forth in SEQ ID NO: 9, 10 or 11, wherein said sequence is modified by addition, deletion or substitution of one or more amino acid sequences and wherein said polypeptide has reduced or abrogated enzyme activity in the conversion of (*S*)-reticuline to 1,2-dehydroreticuline and/or to (*R*)-reticuline when compared to the unmodified polypeptide comprising the amino acid sequence set forth in SEQ ID NO: 9, 10 or 11.

5. The plant according to any one of claims 1 to 4 wherein said modified polypeptide has reduced or abrogated activity and is selected from the group consisting of:
i) a modified polypeptide comprising a modified amino acid sequence of the amino acid sequence set forth in SEQ ID NO: 15;
ii) a modified polypeptide comprising a modified amino acid sequence of the amino acid sequence set forth in SEQ ID NO: 16;
ii) a modified polypeptide comprising a modified amino acid sequence that is at least 95% identical to the full length amino acid sequence as set forth in SEQ ID NO: 15 or 16, wherein said sequence is modified by addition, deletion or substitution of one or more amino acid sequences and wherein said polypeptide has reduced or abrogated enzyme activity in the conversion of 1,2-dehydroreticuline to (*R*)-reticuline when compared to the unmodified polypeptide comprising the amino acid sequence set forth in SEQ ID NO: 15 or 16.

6. The plant according to claim 4 wherein said modified polypeptide is selected from the group:
i) at least an addition, deletion or substitution of an amino acid residue in the region of amino acids 386-486 as set forth in SEQ ID NO: 9, 10 or 11;
ii) at least an addition, deletion or substitution of an amino acid residue in the region of amino acids 426-436 as set forth in SEQ ID NO: 9, 10 or 11 and wherein said modified polypeptide is at least 90% identical to the amino acid sequence set forth in SEQ ID NO: 9, 10 or 11; or
iii) said modified polypeptide comprises a deletion or substitution of amino acid residue 431 of SEQ ID NO: 9 wherein said modified polypeptide is at least 90% identical to the amino acid sequence set forth in SEQ ID NO: 9.
iv) said modified polypeptide comprises a deletion or substitution of amino acid residue 432 of SEQ ID NO: 10 wherein said modified polypeptide is at least 90% identical to the amino acid sequence set forth in SEQ ID NO: 10;
v) said modified polypeptide comprises a deletion or substitution of amino acid residue 431 of SEQ ID NO: 11 wherein said modified polypeptide is at least 90% identical to the amino acid sequence set forth in SEQ ID NO: 11;
vi) said modified polypeptide comprises a substitution or deletion at amino acid arginine 431 of the amino acid sequence set forth in SEQ ID NO: 9;
vii) wherein said modified polypeptide comprises a substitution or deletion at amino acid arginine 432 of the amino acid sequence set forth in SEQ ID NO: 10;
viii) wherein said modified polypeptide comprises a substitution or deletion at amino acid arginine 431 of the amino acid sequence set forth in SEQ ID NO: 11;
ix) wherein said modification is the substitution of arginine for tryptophan in at position 431 of the amino acid sequences set forth in SEQ ID NO: 9;
x) wherein said modification is the substitution of arginine for tryptophan in at position 432 of the amino acid sequences set forth in SEQ ID NO: 10;
xi) wherein said modification is the substitution of arginine for tryptophan in at position 431 of the amino acid sequences set forth in SEQ ID NO: 11.

7. The plant according to claim 4 wherein said modified polypeptide comprises the amino acid sequence set forth in SEQ ID NO: 27, 28 or29.

8. The plant according to any one of claims 1 to 7 wherein said plant comprises one or more genes comprising a nucleotide sequence selected from the group consisting of:
i) a nucleotide sequence as represented by the sequence in SEQ ID NO: 17,18, 19, 20, 21, 22, 23, 24, 25 or 26;
ii) a nucleotide sequence wherein said sequence is degenerate as a result of the genetic code to the nucleotide sequence defined in (i);
iii) a nucleic acid molecule the complementary strand of which hybridizes under stringent hybridization conditions to the sequence in SEQ ID NO: 17,18, 19, 20, 21, 22, 23, 24, 25 or 26 wherein said nucleic acid molecule encodes polypeptides involved in the biosynthesis of *Papaver* noscapine or intermediates in the biosynthesis of noscapine.

9. The plant according to any one of claims 1 to 8 wherein the plant is null for said cytochrome P450-oxidoreductase.

10. The plant according to any one of claims 1 to 9 wherein the plant is heterozygous for said modified gene.

11. The plant according to any one of claims 1 to 9 wherein the plant is homozygous for said modified gene.

12. The plant according to any one of claims 1 to 11 wherein the plant is *Papaver somniferum.*

13. A Papaver capsule, straw or seed obtained from the modified plant according to any one of claims 1 to 12.

14. The use of a gene encoding a cytochrome P450-oxidoreductase catalysing the conversion of (*S*)-reticuline to (*R*)-reticuline in the identification of *Papaver* cultivars that have enhanced levels of noscapine or intermediates in noscapine synthesis wherein said gene comprises a nucleotide sequence selected from the group consisting of:
i) a nucleic acid molecule comprising a nucleic acid sequence as set forth in SEQ ID NO: 6, 7 or 8; or
ii) a nucleic acid molecule the complementary strand of which hybridizes under stringent hybridization conditions to the nucleotide sequence set forth in SEQ ID NO: 6, 7 or, 8 and which encodes a polypeptide that has reduced or abrogated expression or activity of a cytochrome P450-oxidoreductase catalysing the conversion of (*S*)-reticuline to (*R*)-reticuline compared to a wild-type *Papaver* plant comprising a gene encoding a functional cytochrome P450-oxidoreductase.

15. A method to identify a *Papaver* plant comprising a modified cytochrome P450-oxidoreductase gene comprising the steps:
i) obtaining a sample of plant material from a plant to be tested;
ii) extracting genomic nucleic acid from said plant material; and
iii) analysing the extracted genomic nucleic acid encoding a variant nucleotide sequence of the nucleotide sequences set forth in SEQ ID NO: 6, 7 or 8; and optionally,
iv) comparing the sequence to a nucleotide sequence comprising SEQ ID NO: 6, 7 or 8; and optionally
v) preparing a reaction mixture comprising:
a) genomic DNA to be analysed,
b) a first allele specific forward oligonucleotide primer comprising a nucleotide sequence complementary to said cytochrome P450-oxidoreductase gene nucleotide sequence and a nucleotide sequence complementary to a nucleotide sequence of a first fluorescent reporter cassette,
c) a second allele specific forward oligonucleotide primer comprising a nucleotide sequence complementary to said cytochrome P450-oxidoreductase gene an a nucleotide sequence complementary to a nucleotide sequence of a second fluorescent reporter cassette wherein the nucleotide at the 3'-end of said second oligonucleotide primer sequence is complimentary to a polymorphic nucleotide sequence variant of said cytochrome P450-oxidoreductase gene, and wherein the nucleotide sequence of said first and second oligonucleotide primers are complementary to the same upstream nucleotide sequence of said cytochrome P450-oxidoreductase gene;
d) a reverse oligonucleotide primer comprising a nucleotide sequence complementary to a nucleotide sequence downstream of said cytochrome P450-oxidoreductase gene nucleotide sequence; and
e) two reporter cassettes comprising double stranded nucleic acid comprising:
a first double stranded nucleic acid wherein one strand comprises a fluorescent label and is complementary to said first allele specific primer and a second strand comprising a quencher label that quenches said fluorescent label; and
a second double stranded nucleic acid wherein one strand comprises a fluorescent label different from the fluorescent label of i) and is complementary to said second allele specific primer and a second strand comprising a quencher label that quenches said second fluorescent label;
ii) heating said reaction mixture to denature genomic nucleic acid and said double stranded reporters and providing conditions to anneal primers to their respective complementary nucleotide sequence, primer extending said annealed oligonucleotide primers and polymerase chain amplification of said mixture; and
iii) detecting fluorescence emission.

16. A method for the extraction of noscapine from a modified plant or capsule or straw according to the invention comprising the steps:
i) harvesting a modified plant, capsule or straw according any one of claims 1 to 13 and forming a reaction mixture of particulate plant material;
ii) solvent extracting said reaction mixture to provide an alkaloid enriched fraction; and
iii) concentrating said enriched alkaloid fraction to provide an enriched noscapine fraction.

## Patentansprüche

1. Pflanze der Gattung *Papaver,* wobei die Pflanze in einem Gen modifiziert ist, umfassend eine Nukleotidsequenz wie dargelegt in SEQ ID NO: 6, 7 oder 8 oder ein Nukleinsäuremolekül, das über die gesamte Nukleotidsequenz zu mindestens 90% mit der in SEQ ID NO 6, 7 oder 8 dargelegten Nukleotidsequenz identisch ist und für eine modifizierte Cytochrom P450-Oxidoreduktase kodiert, die die Umwandlung von (S)-Reticulin in (R)-Reticulin katalysiert, wobei die Modifikation homozygot ist, stabil vererbt wird und die Expression oder Aktivität der Domäne Cytochrom P450 und/oder der Oxidoreduktase-Domäne des modifizierten Polypeptids verringert oder aufhebt, wobei Noscapin mindestens 50% des Benzylisochinolinalkaloid-Gehalts der modifizierten Pflanze umfasst.

2. Pflanze nach Anspruch 1, wobei das modifizierte Nukleinsäuremolekül wie folgt ist:
i) zu mindestens 91%, 92%, 93%, 94%, 95%, 96%, 97%, 98% oder 99% über die gesamte Nukleotidsequenz identisch mit der Nukleotidsequenz, die in SEQ ID NO: 6, 7 oder 8 dargelegt ist; oder
ii) zu mindestens 99% über die gesamte Nukleotidsequenz identisch mit der Nukleotidsequenz, die in SEQ ID NO 6, 7 oder 8 dargelegt ist, und wobei die Modifikation eine Addition, Substitution oder Deletion von mindestens einer Nukleotidbase zwischen den Nukleotiden 1280-1300 ist, die in SEQ ID NO 6, 7 oder 8 dargelegt sind.

3. Pflanze nach Anspruch 2, wobei die Modifikation eine Substitution mindestens einer Nukleotidbase zwischen den Nukleotiden 1280-1300 ist, die in SEQ ID NO 6, 7 oder 8 dargelegt sind, oder wobei die Modifikation eine Substitution mindestens einer Nukleotidbase zwischen den Nukleotiden 1285-1295 ist, die in SEQ ID NO 6, 7 oder 8 dargelegt sind, oder wobei die Modifikation eine C-zu-T-Substitution der Nukleotidbase an dem Nukleotid 1291 von SEQ ID NO 6, 7 oder 8 ist.

4. Pflanze nach einem der Ansprüche 1 bis 3, wobei das modifizierte Polypeptid eine verringerte oder aufgehobene Aktivität aufweist und ausgewählt ist aus der Gruppe, bestehend aus:
i) einem Polypeptid, umfassend eine modifizierte Aminosäuresequenz der Aminosäuresequenz, die in SEQ ID NO: 9, 10 oder 11 dargelegt ist;
ii) einem Polypeptid, umfassend eine modifizierte Aminosäuresequenz, die zu mindestens 95% identisch ist mit der Aminosäuresequenz in gesamter Länge, wie sie in SEQ ID NO: 9, 10 oder 11 dargelegt ist, wobei die Sequenz durch Addition, Deletion oder Substitution einer oder mehrerer Aminosäuresequenzen modifiziert ist und wobei das Polypeptid eine verringerte oder aufgehobene Enzymaktivität bei der Umwandlung von (*S*)-Reticulin in 1,2-Dehydroreticulin und/oder in (*R*)-Reticulin im Vergleich zu dem unmodifizierten Polypeptid aufweist, umfassend die Aminosäuresequenz, die in SEQ ID NO: 9, 10 oder 11 dargelegt ist.

5. Pflanze nach einem der Ansprüche 1 bis 4, wobei das modifizierte Polypeptid eine verringerte oder aufgehobene Aktivität aufweist und ausgewählt ist aus der Gruppe, bestehend aus:
i) einem modifizierten Polypeptid, umfassend eine modifizierte Aminosäuresequenz der Aminosäuresequenz, die in SEQ ID NO: 15 dargelegt ist;
ii) einem modifizierten Polypeptid, umfassend eine modifizierte Aminosäuresequenz der Aminosäuresequenz, die in SEQ ID NO: 16 dargelegt ist;
ii) einem modifizierten Polypeptid, umfassend eine modifizierte Aminosäuresequenz, die zu mindestens 95% identisch ist mit der Aminosäuresequenz in gesamter Länge, wie sie in SEQ ID NO: 15 oder 16 dargelegt ist, wobei die Sequenz durch Addition, Deletion oder Substitution einer oder mehrerer Aminosäuresequenzen modifiziert ist und wobei das Polypeptid eine verringerte oder aufgehobene Enzymaktivität bei der Umwandlung von 1,2-Dehydroreticulin in (*R*)-Reticulin im Vergleich zu dem unmodifizierten Polypeptid aufweist, umfassend die Aminosäuresequenz, die in SEQ ID NO: 15 oder 16 dargelegt ist.

6. Pflanze nach Anspruch 4, wobei das modifizierte Polypeptid ausgewählt ist aus der folgenden Gruppe:
i) mindestens eine Addition, Deletion oder Substitution eines Aminosäurerests in der Region von Aminosäuren 386-486, wie sie in SEQ ID NO: 9, 10 oder 11 dargelegt sind;
ii) mindestens eine Addition, Deletion oder Substitution eines Aminosäurerests in der Region von Aminosäuren 426-436, wie sie in SEQ ID NO: 9, 10 oder 11 dargelegt sind, und wobei das modifizierte Polypeptid zu mindestens 90% identisch mit der Aminosäuresequenz ist, die in SEQ ID NO: 9, 10 oder 11 dargelegt ist; oder
iii) das modifizierte Polypeptid umfasst eine Deletion oder Substitution von Aminosäurerest 431 von SEQ ID NO: 9, wobei das modifizierte Polypeptid zu mindestens 90% identisch mit der Aminosäuresequenz ist, die in SEQ ID NO: 9 dargelegt ist
iv) das modifizierte Polypeptid umfasst eine Deletion oder Substitution von Aminosäurerest 432 von SEQ ID NO: 10, wobei das modifizierte Polypeptid zu mindestens 90% identisch mit der Aminosäuresequenz ist, die in SEQ ID NO: 10 dargelegt ist;
v) das modifizierte Polypeptid umfasst eine Deletion oder Substitution von Aminosäurerest 431 von SEQ ID NO: 11, wobei das modifizierte Polypeptid zu mindestens 90% identisch mit der Aminosäuresequenz ist, die in SEQ ID NO: 11 dargelegt ist;
vi) das modifizierte Polypeptid umfasst eine Substitution oder Deletion an der Aminosäure Arginin 431 der Aminosäuresequenz, die in SEQ ID NO: 9 dargelegt ist;
vii) wobei das modifizierte Polypeptid eine Substitution oder Deletion an der Aminosäure Arginin 432 der Aminosäuresequenz umfasst, die in SEQ ID NO: 10 dargelegt ist;
viii) wobei das modifizierte Polypeptid eine Substitution oder Deletion an der Aminosäure Arginin 431 der Aminosäuresequenz umfasst, die in SEQ ID NO: 11 dargelegt ist;
ix) wobei die Modifikation die Substitution von Arginin durch Tryptophan an Position 431 der Aminosäuresequenzen ist, die in SEQ ID NO: 9 dargelegt sind;
x) wobei die Modifikation die Substitution von Arginin durch Tryptophan an Position 432 der Aminosäuresequenzen ist, die in SEQ ID NO: 10 dargelegt sind;
xi) wobei die Modifikation die Substitution von Arginin durch Tryptophan an Position 431 der Aminosäuresequenzen ist, die in SEQ ID NO: 11 dargelegt sind.

7. Pflanze nach Anspruch 4, wobei das modifizierte Polypeptid die Aminosäuresequenz umfasst, die in SEQ ID NO: 27, 28 oder 29 dargelegt ist.

8. Pflanze nach einem der Ansprüche 1 bis 7, wobei die Pflanze ein oder mehrere Gene umfasst, umfassend eine Nukleotidsequenz, die ausgewählt ist aus der Gruppe, bestehend aus:
i) einer Nukleotidsequenz, die durch die Sequenz in SEQ ID NO: 17, 18, 19, 20, 21, 22, 23, 24, 25 oder 26 dargestellt ist;
ii) einer Nukleotidsequenz, wobei die Sequenz als Resultat des genetischen Codes zu der Nukleotidsequenz degeneriert ist, die in (i) definiert ist;
iii) einem Nukleinsäuremolekül, dessen komplementärer Strang unter stringenten Hybridisierungsbedingungen mit der Sequenz in SEQ ID NO: 17, 18, 19, 20, 21, 22, 23, 24, 25 oder 26 hybridisiert, wobei das Nukleinsäuremolekül für Polypeptide kodiert, die bei der Biosynthese von *Papaver*-Noscapin oder Zwischenprodukten bei der Biosynthese von Noscapin involviert sind.

9. Pflanze nach einem der Ansprüche 1 bis 8, wobei die Pflanze nichtig für die Cytochrom-P450-Oxidoreduktase ist.

10. Pflanze nach einem der Ansprüche 1 bis 9, wobei die Pflanze heterozygot für das modifizierte Gen ist.

11. Pflanze nach einem der Ansprüche 1 bis 9, wobei die Pflanze homozygot für das modifizierte Gen ist.

12. Pflanze nach einem der Ansprüche 1 bis 11, wobei die Pflanze *Papaver somniferum* ist.

13. Papaver-Kapsel, -Stroh oder -Samen, erlangt aus der modifizierten Pflanze nach einem der Ansprüche 1 bis 12.

14. Verwendung eines Gens, das für eine Cytochrom-P450-Oxidoreduktase kodiert, die die Umwandlung von (*S*)-Reticulin in (*R*)-Reticulin katalysiert, bei der Identifizierung von *Papaver-*Kultivaren, die erhöhte Pegel an Noscapin oder Zwischenprodukten bei der Noscapin-Synthese aufweisen, wobei das Gen eine Nukleotidsequenz umfasst, die ausgewählt ist aus der Gruppe, bestehend aus:
i) einem Nukleinsäuremolekül, umfassend eine Nukleinsäuresequenz wie dargelegt in SEQ ID NO: 6, 7 oder 8; oder
ii) einem Nukleinsäuremolekül, dessen komplementärer Strang unter stringenten Hybridisierungsbedingungen mit der Nukleotidsequenz hybridisiert, die in SEQ ID NO: 6, 7 oder 8 dargelegt ist, und das für ein Polypeptid kodiert, das eine verringerte oder aufgehobene Expression oder Aktivität einer Cytochrom-P450-Oxidoreduktase aufweist, die die Umwandlung von (*S*)-Reticulin in (*R*)-Reticulin katalysiert, im Vergleich zu einer Wildtyp-*Papaver*-Pflanze, umfassend ein Gen, das für eine funktionelle Cytochrom-P450-Oxidoreduktase kodiert.

15. Verfahren zum Identifizieren einer *Papaver*-Pflanze, umfassend ein modifiziertes Cytochrom-P450-Oxidoreduktase-Gen, umfassend die folgenden Schritte:
i) Erlangen einer Probe von Pflanzenmaterial von einer zu prüfenden Pflanze,
ii) Extrahieren von genomischer Nukleinsäure aus dem Pflanzenmaterial; und
iii) Analysieren der extrahierten genomischen Nukleinsäure, die für eine Nukleotidsequenz-Variante der Nukleotidsequenzen kodiert, die in SEQ ID NO: 6, 7 oder 8 dargelegt sind; und optional
iv) Vergleichen der Sequenz mit einer Nukleotidsequenz, umfassend SEQ ID NO: 6, 7 oder 8; und optional
v) Zubereiten eines Reaktionsgemischs, umfassend:
a) genomische DNA, die analysiert werden soll,
b) einen ersten Allel-spezifischen Vorwärts-Oligonukleotid-Primer, umfassend eine Nukleotidsequenz, die komplementär zu der Nukleotidsequenz des Cytochrom-P450-Oxidoreduktase-Gens ist, und eine Nukleotidsequenz, die komplementär zu einer Nukleotidsequenz einer ersten fluoreszierenden Reporterkassette ist,
c) einen zweiten Allel-spezifischen Vorwärts-Oligonukleotid-Primer, umfassend eine Nukleotidsequenz, die komplementär zu dem Cytochrom-P450-Oxidoreduktase-Gen ist, und eine Nukleotidsequenz, die komplementär zu einer Nukleotidsequenz einer zweiten fluoreszierenden Reporterkassette ist, wobei das Nukleotid an dem 3'-Ende der zweiten Oligonukleotid-Primersequenz komplementär zu einer polymorphen Nukleotidsequenz-Variante des Cytochrom-P450-Oxidoreduktase-Gens ist, und wobei die Nukleotidsequenz des ersten und des zweiten Oligonukleotid-Primers komplementär zu der gleichen stromaufwärtigen Nukleotidsequenz des Cytochrom-P450-Oxidoreduktase-Gens ist;
d) einen reversen Oligonukleotid-Primer, umfassend eine Nukleotidsequenz, die komplementär zu einer Nukleotidsequenz stromabwärts von der Nukleotidsequenz des Cytochrom-P450-Oxidoreduktase-Gens ist; und
e) zwei Reporterkassetten, umfassend doppelsträngige Nukleinsäure, umfassend:
eine erste doppelsträngige Nukleinsäure, wobei ein Strang eine fluoreszierende Markierung umfasst und komplementär zu dem ersten Allel-spezifischen Primer ist und ein zweiter Strang eine Quencher-Markierung umfasst, die die fluoreszierende Markierung quencht; und
eine zweite doppelsträngige Nukleinsäure, wobei ein Strang eine fluoreszierende Markierung umfasst, die sich von der fluoreszierenden Markierung von i) unterscheidet und komplementär zu dem zweiten Allel-spezifischen Primer ist, und einen zweiten Strang, der eine Quencher-Markierung umfasst, die die zweite fluoreszierende Markierung quencht;
ii) Erhitzen des Reaktionsgemischs, um die genomische Nukleinsäure und die doppelsträngigen Reporter zu denaturieren, und Bereitstellen von Bedingungen, um die Primer an ihre jeweilige komplementäre Nukleotidsequenz anzulagern, Primer, der die angelagerten Oligonukleotid-Primer erweitert, und Polymerasekettenamplifikation des Gemischs; und
iii) Detektion von Fluoreszenzemission.

16. Verfahren zur Extraktion von Noscapin aus einer modifizierten Pflanze oder Kapsel oder Stroh nach der Erfindung, umfassend die folgenden Schritte:
i) Ernten einer modifizierten Pflanze, Kapsel oder eines Strohs nach einem der Ansprüche 1 bis 13 und Bilden eines Reaktionsgemischs aus teilchenförmigem Pflanzenmaterial;
ii) Extrahieren mit Lösungsmittel des Reaktionsgemischs, um eine alkaloidangereicherte Fraktion bereitzustellen; und
iii) Konzentrieren der angereicherten Alkaloidfraktion, um eine angereicherte Noscapinfraktion bereitzustellen.

## Revendications

1. Plante du genre *Papaver,* ladite plante étant modifiée dans un gène comprenant une séquence nucléotidique telle que décrite dans SEQ ID n° : 6, 7 ou 8, ou une molécule d'acide nucléique qui est identique à au moins 90 % à la séquence nucléotidique décrite dans SEQ ID n° : 6, 7 ou 8 sur la séquence nucléotidique complète et code un cytochrome P450-oxydoréductase modifiée catalysant la conversion de la (*S*)-réticuline en (*R*)-réticuline dans laquelle ladite modification est homozygote, héritée de façon stable et réduit ou supprime l'expression ou l'activité dudit domaine cytochrome P450 et/ou domaine oxydoréductase dudit polypeptide modifié dans lequel la noscapine comprend au moins 50 % de la teneur en alcaloïdes de benzylisoquinoline de ladite plante modifiée.

2. Plante selon la revendication 1, ladite molécule d'acide nucléique modifiée étant :
i) identique à au moins 91 %, 92 %, 93 %, 94 %, 95 %, 96 %, 97 %, 98 % ou 99 % à la séquence nucléotidique décrite dans SEQ ID n° : 6, 7 ou 8 sur la séquence nucléotidique complète ; ou
ii) étant identique à au moins 99 % à la séquence nucléotidique décrite dans SEQ ID n° : 6, 7 ou 8, sur la séquence nucléotidique complète et ladite modification étant une addition, une substitution ou une délétion d'au moins une base nucléotidique entre les nucléotides 1280 à 1300 décrite dans SEQ ID n° : 6, 7 ou 8.

3. Plante selon la revendication 2, ladite modification étant une substitution d'au moins une base nucléotidique entre les nucléotides 1280 à 1300 décrite dans SEQ ID n° : 6, 7 ou 8 ou ladite modification étant une substitution d'au moins une base nucléotidique entre les nucléotides 1285 à 1295 décrite dans SEQ ID n° : 6, 7 ou 8 ou ladite modification étant une substitution de C en T de la base nucléotidique au niveau du nucléotide 1291 de SEQ ID n° : 6, 7 ou 8.

4. Plante selon l'une quelconque des revendications 1 à 3, ledit polypeptide modifié comportant une activité réduite ou supprimée et étant choisi dans le groupe constitué par :
i) un polypeptide comprenant une séquence d'acides aminés modifiée de la séquence d'acides aminés décrite dans SEQ ID n° : 9, 10 ou 11 ;
ii) un polypeptide comprenant une séquence d'acides aminés modifiée qui est identique à au moins 95 % à la séquence d'acides aminés pleine longueur telle que décrite dans SEQ ID n° : 9, 10 ou 11, ladite séquence étant modifiée par l'addition, la délétion ou la substitution d'une ou plusieurs séquences d'acides aminés et ledit polypeptide comportant une activité enzymatique réduite ou supprimée dans la conversion de la (*S*)-réticuline en 1,2-déshydroréticuline et/ou en (R)-réticuline lorsqu'il est comparé au polypeptide non modifié comprenant la séquence d'acides aminés décrite dans SEQ ID n° : 9, 10 ou 11.

5. Plante selon l'une quelconque des revendications 1 à 4, ledit polypeptide modifié comportant une activité réduite ou supprimée et étant choisi dans le groupe constitué par :
i) un polypeptide comprenant une séquence d'acides aminés modifiée de la séquence d'acides aminés décrite dans SEQ ID n° : 15 ;
ii) un polypeptide comprenant une séquence d'acides aminés modifiée de la séquence d'acides aminés décrite dans SEQ ID n° : 16 ;
iii) un polypeptide comprenant une séquence d'acides aminés modifiée qui est identique à au moins 95 % à la séquence d'acides aminés pleine longueur telle que décrite dans SEQ ID n° : 15 ou 16, ladite séquence étant modifiée par l'addition, la délétion ou la substitution d'une ou plusieurs séquences d'acides aminés et ledit polypeptide comportant une activité enzymatique réduite ou supprimée dans la conversion de la 1,2-déshydroréticuline en (*R*)-réticuline lorsqu'il est comparé au polypeptide non modifié comprenant la séquence d'acides aminés décrite dans SEQ ID n° : 15 ou 16.

6. Plante selon la revendication 4, ledit polypeptide modifié étant choisi dans le groupe :
i) au moins une addition, délétion ou substitution d'un résidu d'acide aminé dans la région des acides aminés 386 à 486 telle que décrite dans SEQ ID n° : 9, 10 ou 11 ;
ii) au moins une addition, délétion ou substitution d'un résidu d'acide aminé dans la région des acides aminés 426 à 436 telle que décrite dans SEQ ID n° : 9, 10 ou 11 et ledit polypeptide modifié étant identique à au moins 90 % à la séquence d'acides aminés décrite dans SEQ ID n° : 9, 10 ou 11 ; ou
iii) ledit polypeptide modifié comprend une délétion ou substitution de résidu d'acide aminé 431 de SEQ ID n° : 9, ledit polypeptide modifié étant identique à au moins 90 % à la séquence d'acides aminés décrite dans SEQ ID n° : 9 ;
iv) ledit polypeptide modifié comprend une délétion ou substitution de résidu d'acide aminé 432 de SEQ ID n° : 10, ledit polypeptide modifié étant identique à au moins 90 % à la séquence d'acides aminés décrite dans SEQ ID n° : 10 ;
v) ledit polypeptide modifié comprend une délétion ou substitution de résidu d'acide aminé 431 de SEQ ID n° : 11, ledit polypeptide modifié étant identique à au moins 90 % à la séquence d'acides aminés décrite dans SEQ ID n° : 11 ;
vi) ledit polypeptide modifié comprend une substitution ou délétion au niveau de l'acide aminé arginine 431 de la séquence d'acides aminés décrite dans SEQ ID n° : 9 ;
vii) ledit polypeptide modifié comprend une substitution ou délétion au niveau de l'acide aminé arginine 432 de la séquence d'acides aminés décrite dans SEQ ID n° : 10 ;
viii) ledit polypeptide modifié comprend une substitution ou délétion au niveau de l'acide aminé arginine 431 de la séquence d'acides aminés décrite dans SEQ ID n° : 11 ;
ix) ladite modification étant la substitution de l'arginine par le tryptophane au niveau de la position 431 des séquences d'acides aminés décrites dans SEQ ID n° : 9 ;
x) ladite modification étant la substitution de l'arginine par le tryptophane au niveau de la position 432 des séquences d'acides aminés décrites dans SEQ ID n° : 10 ;
xi) ladite modification étant la substitution de l'arginine par le tryptophane au niveau de la position 431 des séquences d'acides aminés décrites dans SEQ ID n° : 11.

7. Plante selon la revendication 4, ledit polypeptide modifié comprend la séquence d'acides aminés décrite dans SEQ ID n° : 27, 28 ou 29.

8. Plante selon l'une quelconque des revendications 1 à 7, ladite plante comprend un ou plusieurs gènes comprenant une séquence nucléotidique choisie dans le groupe constitué par :
i) une séquence nucléotidique telle que représentée par la séquence dans SEQ ID n° : 17, 18, 19, 20, 21, 22, 23, 24, 25 ou 26 ;
ii) une séquence nucléotidique, ladite séquence étant dégénérée en raison du code génétique en la séquence nucléotidique définie en (i) ;
iii) une molécule d'acide nucléique dont le brin s'hybride dans des conditions d'hybridation stringentes à la séquence dans SEQ ID n° : 17, 18, 19, 20, 21, 22, 23, 24, 25 ou 26, ladite molécule d'acide nucléique codant des polypeptides impliqués dans la biosynthèse de la noscapine de *Papaver* ou des intermédiaires dans la biosynthèse de la noscapine.

9. Plante selon l'une quelconque des revendications 1 à 8, ladite plante étant nulle pour ledit cytochrome P450-oxydoréductase.

10. Plante selon l'une quelconque des revendications 1 à 9, ladite plante étant hétérozygote pour ledit gène modifié.

11. Plante selon l'une quelconque des revendications 1 à 9, ladite plante étant homozygote pour ledit gène modifié.

12. Plante selon l'une quelconque des revendications 1 à 11, ladite plante étant *Papaver somniferum.*

13. Capsule, paille ou graine de *Papaver* obtenue à partir de la plante modifiée selon l'une quelconque des revendications 1 à 12.

14. Utilisation d'un gène codant un cytochrome P450-oxydoréductase catalysant la conversion de la (*S*)-réticuline en (*R*)-réticuline dans l'identification de cultivars de *Papaver* qui comportent des taux améliorés de noscapine ou d'intermédiaires dans la synthèse de noscapine, ledit gène comprenant une séquence nucléotidique choisie dans le groupe constitué par :
i) une molécule d'acide nucléique comprenant une séquence d'acides nucléiques décrite dans SEQ ID n° : 6, 7 ou 8 ; ou
ii) une molécule d'acide nucléique dont le brin complémentaire s'hybride dans des conditions d'hybridation stringentes à la séquence nucléotidique décrite dans SEQ ID n° : 6, 7 ou 8 et qui code un polypeptide qui comporte une expression ou activité réduite ou supprimée d'un cytochrome P450-oxydoréductase catalysant la conversion de la (*S*)-réticuline en (*R*)-réticuline par rapport à la plante *Papaver* de type sauvage comprenant un gène codant un cytochrome P450-oxydoréductase fonctionnel.

15. Méthode d'identification d'une plante *Papaver* comprenant un gène de cytochrome P450-oxydoréductase modifié comprenant les étapes :
i) obtention d'un échantillon de matière végétale d'une plante à tester ;
ii) extraction d'acide nucléique génomique de ladite matière végétale ; et
iii) analyse de l'acide nucléique génomique extrait codant une séquence nucléotidique variante des séquences nucléotidiques décrites dans SEQ ID n° : 6, 7 ou 8 ; et éventuellement
iv) comparaison de la séquence à une séquence nucléotidique comprenant SEQ ID n° : 6, 7 ou 8 ; et éventuellement
v) préparation d'un mélange réactionnel comprenant :
a) de l'ADN génomique à analyser,
b) une première amorce oligonucléotidique avant spécifique à un allèle comprenant une séquence nucléotidique complémentaire à ladite séquence nucléotidique du gène de cytochrome P450-oxydoréductase et une séquence nucléotidique complémentaire à une séquence nucléotidique d'une première cassette de rapporteur fluorescent,
c) une seconde amorce oligonucléotidique avant spécifique à un allèle comprenant une séquence nucléotidique complémentaire audit gène de cytochrome P450-oxydoréductase et une séquence nucléotidique complémentaire à une séquence nucléotidique d'une seconde cassette de rapporteur fluorescent dans laquelle le nucléotide au niveau de l'extrémité 3' de ladite séquence de seconde amorce nucléotidique est complémentaire à un variant de séquence nucléotidique polymorphique dudit gène de cytochrome P450-oxydoréductase, et ladite séquence nucléotidique desdites premières et secondes amorces oligonucléotidiques étant complémentaires à la même séquence nucléotidique en amont dudit gène de cytochrome P450-oxydoréductase ;
d) une amorce oligonucléotidique inverse comprenant une séquence nucléotidique complémentaire à une séquence nucléotidique en aval de ladite séquence nucléotidique du gène de cytochrome P450-oxydoréductase ; et
e) deux cassettes de rapporteur comprenant un acide nucléique double brin comprenant :
un premier acide nucléique double brin dans lequel un brin comprend un marqueur fluorescent et est complémentaire à ladite première amorce spécifique à un allèle et un second brin comprenant un marqueur extincteur qui éteint ledit marqueur fluorescent ; et
un second acide nucléique double brin dans lequel un brin comprend un marqueur fluorescent différent du marqueur fluorescent de i) et est complémentaire à ladite seconde amorce spécifique à un allèle et un second brin comprenant un marqueur extincteur qui éteint ledit second marqueur fluorescent ;
ii) chauffage dudit mélange réactionnel pour dénaturer l'acide nucléique génomique et lesdits rapporteurs double brin et fourniture de conditions pour hybrider les amorces à leur séquence nucléotidique complémentaire respective, une amorce étendant lesdites amorces oligonucléotidiques hybridées et amplification en chaîne par polymérase dudit mélange ; et
iii) détection de l'émission de fluorescence.

16. Méthode permettant l'extraction de la noscapine d'une plante ou capsule ou paille modifiée selon l'invention comprenant les étapes :
i) récolte d'une plante ou capsule ou paille modifiée selon l'une quelconque des revendications 1 à 13 et formation d'un mélange réactionnel de matière végétale particulaire ;
ii) extraction au solvant dudit mélange réactionnel pour fournir une fraction enrichie en alcaloïdes ; et
iii) concentration de ladite fraction d'alcaloïdes enrichie pour fournir une fraction de noscapine enrichie.
